(19) **Europäisches Patentamt / European Patent Office / Office européen des brevets**

(11) **EP 3 486 712 B1**

(12) **FASCICULE DE BREVET EUROPEEN**

(45) Date de publication et mention de la délivrance du brevet:
**23.04.2025   Bulletin 2025/17**

(21) Numéro de dépôt: **18213420.5**

(22) Date de dépôt: **16.05.2013**

(51) Classification Internationale des Brevets (IPC):
**G02C 7/10** $^{(2006.01)}$     **G02B 5/28** $^{(2006.01)}$
**G02B 1/11** $^{(2015.01)}$     **G02C 7/02** $^{(2006.01)}$
**G02B 5/26** $^{(2006.01)}$

(52) Classification Coopérative des Brevets (CPC):
**G02C 7/107; G02B 1/11; G02B 5/26; G02B 5/283; G02C 7/02; G02C 7/104**

(54) **LENTILLE OPHTALMIQUE**

**KONTAKTLINSE**

**OPHTHALMIC LENS**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:   **16.05.2012   FR 1254529**
   **11.10.2012   FR 1259713**

(43) Date de publication de la demande:
**22.05.2019   Bulletin 2019/21**

(62) Numéro(s) de document de la (des) demande(s) initiale(s) en application de l'article 76 CBE:
**13729971.5 / 2 850 484**

(73) Titulaire: **Essilor International**
**94220 Charenton-Le-Pont (FR)**

(72) Inventeurs:
• **DE AYGUAVIVES, Francisco**
**94220 Charenton-le-Pont (FR)**
• **MAURY, Hélène**
**94220 Charenton-le-Pont (FR)**

(74) Mandataire: **Jacobacci Coralis Harle**
**32, rue de l'Arcade**
**75008 Paris (FR)**

(56) Documents cités:
WO-A2-2008/024414     US-A- 4 793 669
US-A1- 2004 233 524     US-A1- 2010 149 483

**Description**

L'invention concerne le domaine de l'optique ophtalmique.

Elle concerne plus particulièrement une lentille ophtalmique dont l'une des faces principales comporte un filtre optique destiné à réduire les effets de la phototoxicité de la lumière bleue sur la rétine d'un porteur de lunettes.

Dans l'ensemble de la présente demande de brevet, il sera fait référence à des gammes de valeurs, en particulier de longueurs d'onde et d'angles d'incidence. L'expression « compris entre les valeurs $x$ et $y$ » signifie « dans la gamme de $x$ à $y$ », les bornes $x$ et $y$ étant incluses dans cette gamme.

La lumière visible par l'œil humain s'étend sur un spectre lumineux allant d'une longueur d'onde de 380 nanomètres (nm) à 780 nm environ. La partie de ce spectre, située entre 380 nm et 500 nm environ, correspond à une lumière sensiblement bleue, de haute énergie.

De nombreuses études (voir par exemple Kitchel E., « The effects of blue light on ocular health », Journal of Visual Impairment and Blindness Vol. 94, No. 6, 2000 ou Glazer-Hockstein et al., Retina, Vol. 26, No. 1, pp. 1-4, 2006) suggèrent que la lumière bleue a des effets phototoxiques sur l'œil, et notamment sur la rétine.

En effet, des études de photobiologie oculaire (Algvere P.V. et al., « Age-Related Maculopathy and the Impact of the Blue Light Hazard », Acta Ophthalmo. Scand., Vol. 84, pp. 4-15, 2006) et des études cliniques (Tomany S.C. et al., « Sunlight and the 10-Year Incidence of Age-Related Maculopathy. The Beaver Dam Eye Study », Arch Ophthalmol. , Vol. 122, pp. 750-757, 2004) ont montré qu'une exposition à la lumière bleue trop longue ou trop intense peut induire des pathologies ophtalmiques sévères telles que la dégénérescence maculaire liée à l'âge (DMLA).

Néanmoins, une partie de cette lumière bleue, comprise entre 465 nm et 495 nm environ est bénéfique dans la mesure où elle intervient au niveau des mécanismes de régulation des rythmes biologiques, appelés « cycles circadiens ».

Ainsi, il est recommandé de limiter l'exposition à la lumière bleue potentiellement nocive, en particulier pour la bande de longueur d'onde qui présente une dangerosité accrue (voir notamment tableau B1 de la norme ISO 8980-3 :2003 (E) concernant la fonction de dangerosité de la lumière bleue $B(\lambda)$).

À cet effet, il peut être conseillé de porter devant chacun des yeux une lentille ophtalmique qui empêche ou limite la transmission de la lumière bleue phototoxique jusqu'à la rétine.

Il a déjà été proposé, par exemple dans la demande de brevet WO2008024414, de couper au moins partiellement, la partie gênante du spectre de la lumière bleue de 400 nm à 460 nm au moyen de lentilles comprenant un film inhibant partiellement la lumière dans la gamme de longueurs d'onde souhaitée, par absorption ou par réflexion.

Par ailleurs, l'homme du métier est à la recherche de filtres permettant de minimiser la quantité de lumière bleue nocive reçue par la rétine, tout en permettant de transmettre efficacement la lumière visible pour les longueurs d'onde supérieures à 465 nm afin, d'une part, de conserver une bonne vision pour le porteur et, d'autre part, de ne pas altérer les cycles circadiens.

La difficulté est que les gammes de longueurs d'onde de 420 nm à 450 nm qu'il convient de filtrer sont très proches des gammes de longueurs d'onde qui ne doivent pas, ou très peu, être filtrées.

C'est un objectif de l'invention de fournir une lentille ophtalmique comprenant un filtre par réflexion tenant compte de l'ensemble de l'irradiation lumineuse provenant du milieu environnant et réduisant la lumière bleue reçue par l'œil dans la gamme de longueurs d'onde allant de 420 nm à 450 nm.

Un autre objectif de l'invention est de fournir une lentille ophtalmique comprenant un tel filtre par réflexion qui permet une excellente transmission dans la gamme de longueurs d'onde allant de 465 nm à 495 nm.

Un autre objectif de l'invention est de fournir une lentille ophtalmique comportant un filtre par réflexion présentant les propriétés ci-dessus, qui soit facile et peu coûteux à mettre en œuvre industriellement.

D'une manière générale, il est possible de concevoir des filtres, dits « étroits », très sélectifs, présentant une bande passante limitée et un pic de réflectivité centré sur cette bande passante. Pour limiter la transmission de la lumière bleue phototoxique à la rétine, un filtre étroit adéquat devrait ainsi avoir par exemple une largeur à mi-hauteur de 30 nm entre 420 nm et 450 nm, et un maximum de réflectivité pour la longueur d'onde centrale de 435 nm.

En pratique, des filtres étroits très sélectifs sont généralement constitués d'un empilement comportant un nombre important de couches diélectriques et dont l'épaisseur totale est élevée.

De tels filtres sont longs et coûteux à réaliser industriellement, en particulier lorsque ceux-ci sont déposés sous vide. La multiplication du nombre de couches et la multiplication des interfaces rend également difficile d'atteindre de bonnes propriétés mécaniques.

La prise en compte des contraintes ci-dessus amène à limiter le nombre de couches, ce qui a pour conséquence de limiter les performances en termes de sélectivité spectrale (la largeur à mi-hauteur d'un tel filtre étroit pouvant alors atteindre jusqu'à 70 nm) et angulaire, le filtre devenant faiblement sélectif angulairement. Ceci signifie que si, pour des longueurs d'onde comprises entre 420 nm et 450 nm, la réflectivité de la face principale d'une lentille ophtalmique munie d'un tel filtre étroit est élevée pour des angles d'incidence sur cette face principale compris entre 0° et 15°, alors la réflectivité pour des angles d'incidence compris entre 30° et 45° sur cette même face principale sera également relativement élevée.

L'angle d'incidence est classiquement défini comme l'angle entre la normale à la surface au point d'incidence et la direction du faisceau lumineux contactant cette surface.

Ceci entraîne un certain nombre de conséquences pour un porteur de lunettes équipé de lentilles ophtalmiques sur la face principale avant desquelles a été déposé un filtre optique étroit tel que décrit précédemment. On entendra ici que la face principale avant de la lentille ophtalmique est la face principale de la lentille ophtalmique qui est située le plus loin des yeux du porteur de lunettes. Par opposition, on désignera la face principale de la lentille ophtalmique qui est située le plus près des yeux du porteur la face principale arrière.

Ainsi positionnées par rapport aux yeux du porteur, les lentilles ophtalmiques reçoivent, d'une part, de la lumière « directe » incidente sur les faces principales avant des lentilles ophtalmiques, et, d'autre part, de la lumière « indirecte » provenant de l'arrière du porteur et réfléchie par les lentilles ophtalmiques.

La lumière venant de l'arrière du porteur et réfléchie par la lentille ophtalmique en direction de l'œil du porteur est principalement la lumière incidente sur la face principale arrière de la lentille ophtalmique avec des angles d'incidence compris entre 30° et 45°.

Cette lumière visible provenant de l'arrière du porteur à un angle d'incidence compris entre 30° et 45° traverse la face principale arrière, sur laquelle se produit une première réflexion, puis le substrat pour atteindre la face principale avant comportant ledit filtre.

Par ailleurs, il est connu que les propriétés optiques d'un filtre déposé sur la face principale avant d'une lentille ophtalmique, par exemple la réflectivité, sont équivalentes, que la lumière soit incidente du côté de la face principale avant ou provenant du côté de la face principale arrière.

Si le filtre étroit réfléchit efficacement la lumière bleue dont la longueur d'onde est comprise entre 420 nm et 450 nm pour un angle d'incidence sur la face principale avant compris entre 30° et 45°, alors il réfléchit également efficacement cette lumière bleue arrivant par l'arrière, pour un angle d'incidence compris entre 30° et 45° sur la face principale arrière.

Ainsi, même si la lumière directe incidente sur les faces principales avant des lentilles ophtalmiques est rejetée efficacement grâce à la réflexion sur les filtres étroits déposés sur les faces principales avant, la lumière indirecte provenant de l'arrière du porteur est réfléchie tout aussi efficacement vers les yeux du porteur.

Au final, malgré l'utilisation d'un filtre étroit, la quantité de lumière bleue phototoxique parvenant jusqu'à la rétine du porteur pourra être relativement importante et présenter un danger pour le porteur.

Par ailleurs, le filtre, qu'il soit appliqué en face avant ou arrière se comporte de la même façon vis-à-vis de la lumière dans la gamme de longueur d'ondes de 420 nm à 450 nm, puisque la lentille ophtalmique, dans l'un comme dans l'autre des cas, transmet la lumière dans la gamme de longueurs d'onde de 420 nm à 450 nm. La même conséquence néfaste vis-à-vis de la lumière bleue phototoxique se manifeste donc pour le porteur si le filtre, au lieu d'être appliqué sur la face principale avant de la lentille ophtalmique, est appliqué sur la face principale arrière.

Par ailleurs, comme déjà mentionné précédemment, des filtres par réflexion étroits comportant un nombre restreint de couches et une épaisseur compatible avec une fabrication industrielle à grande échelle ne présentent qu'une sélectivité spectrale réduite, et sont susceptibles de réfléchir une partie significative de la lumière dans la gamme régissant les cycles circadiens.

Afin de répondre aux objectifs de l'invention et de remédier aux inconvénients précités de l'état de la technique, la présente invention propose une lentille ophtalmique munie d'un filtre par réflexion permettant de réduire la quantité de la lumière bleue phototoxique arrivant sur la rétine d'un porteur de cette lentille ophtalmique, tout en préservant au mieux les cycles circadiens.

À cet effet, l'invention concerne une lentille ophtalmique présentant une face principale avant et une face principale arrière, l'une au moins des faces principales comportant un filtre qui confère à la face principale comportant ledit filtre les propriétés suivantes :

- un facteur moyen de réflexion dans le bleu ($R_{m,B}$) sur une gamme de longueurs d'onde allant de 420 nanomètres à 450 nanomètres qui est supérieure ou égale à 5%, pour un angle d'incidence compris entre 0° et 15°,
- une courbe de réflectivité spectrale pour un angle d'incidence compris entre 0° et 15°, cette courbe de réflectivité ayant :

  - un maximum de réflectivité à une longueur d'onde inférieure à 435 nanomètres, et
  - une largeur à mi-hauteur (FWHM) supérieure à 80 nanomètres,

- la valeur de la réflectivité au maximum de réflectivité de la face principale comportant ledit filtre, pour un angle d'incidence de 15°, est au moins 1,5 fois supérieure à la valeur de la réflectivité de cette même face principale, à la longueur d'onde de 435 nm et pour un angle d'incidence de 15°, et
- pour un angle d'incidence $\theta$ compris entre 0° et 15° et pour un angle d'incidence $\theta'$ compris entre 30° et 45°, un paramètre $\Delta(\theta,\theta')$ défini par la relation $\Delta(\theta,\theta') = 1 - [ R_{\theta'}(435\ nm) / R_{\theta}(435\ nm) ]$, tel que ce paramètre $\Delta(\theta,\theta')$ soit supérieur ou égal à 0,6, où

- $R_\theta$(435 nm) représente la valeur de la réflectivité de la face principale comportant ledit filtre, à la longueur d'onde de 435 nanomètres pour l'angle d'incidence $\theta$, et
- $R_{\theta'}$(435 nm) représente la valeur de la réflectivité de la face principale comportant ledit filtre à la longueur d'onde de 435 nanomètres pour l'angle d'incidence $\theta'$.

Dans un autre mode de réalisation, l'invention concerne une lentille ophtalmique présentant une face principale avant et une face principale arrière, l'une au moins des faces principales comportant un filtre qui confère à la face principale comportant ledit filtre les propriétés suivantes :

- un facteur moyen de réflexion dans le bleu ($R_{m,B}$) sur une gamme de longueurs d'onde allant de 420 nanomètres à 450 nanomètres qui est supérieur ou égal à 10 %, pour un angle d'incidence compris entre 0° et 15°,
- une courbe de réflectivité spectrale pour un angle d'incidence compris entre 0° et 15°, cette courbe de réflectivité ayant :

    - un maximum de réflectivité à une longueur d'onde inférieure à 435 nanomètres, et
    - une largeur à mi-hauteur (FWHM) supérieure ou égale à 70 nanomètres, de préférence supérieure ou égale à 75 nm, et

- pour un angle d'incidence $\theta$ compris entre 0° et 15° et pour un angle d'incidence $\theta'$ compris entre 30° et 45°, un paramètre $\Delta(\theta,\theta')$ défini par la relation $\Delta(\theta,\theta') = 1 - [\, R_{\theta'}(435\ nm) / R_\theta(435\ nm)\,]$, tel que ce paramètre $\Delta(\theta,\theta')$ soit supérieur ou égal à 0,5, où

    - $R_\theta$(435 nm) représente la valeur de la réflectivité de la face principale comportant ledit filtre à la longueur d'onde de 435 nanomètres pour l'angle d'incidence $\theta$, et
    - $R_{\theta'}$(435 nm) représente la valeur de la réflectivité de la face principale comportant ledit filtre à la longueur d'onde de 435 nanomètres pour l'angle d'incidence $\theta'$ et

- pour un angle d'incidence compris entre 0° et 15°, un paramètre $\Delta_{spectral}$ défini par la relation $\Delta_{spectral} = 1 - [\, R_{0°\text{-}15°}(480\ nm) / R_{0°\text{-}15°}(435\ nm)\,]$, tel que ce paramètre $\Delta_{spectral}$ soit supérieur ou égal à 0,8, où

    - $R_{0°\text{-}15°}$(480 nm) représente la valeur de la réflectivité de la face principale avant à la longueur d'onde de 480 nanomètres à l'incidence considérée, et

- $R_{0°\text{-}15°}$(435 nm) représente la valeur de la réflectivité de la face principale avant à la longueur d'onde de 435 nanomètres à l'incidence considérée.

Ainsi, la lentille ophtalmique selon l'invention permet de minimiser la transmission de la lumière bleue phototoxique à la rétine d'un porteur de cette lentille ophtalmique, grâce, d'une part, à sa réflectivité moyenne sur une gamme de longueurs d'onde allant de 420 nm à 450 nanomètres et grâce d'autre part, à sa sélectivité angulaire.

En effet, la lentille ophtalmique munie dudit filtre présente, à une longueur d'onde donnée, une réflectivité sensiblement différente pour deux angles d'incidence sur la face principale comportant ledit filtre sensiblement différents.

Par ailleurs, ce filtre est décentré par rapport à la bande de longueurs d'onde de la lumière bleue phototoxique comprise entre 420 nanomètres et 450 nanomètres. En effet, la lentille ophtalmique présente un maximum de réflectivité à une longueur d'onde inférieure à 435 nanomètres. Ceci permet alors d'ajuster la sélectivité angulaire de la lentille.

Les caractéristiques spectrales (réflectivité, $R_m$, $R_v$,..) de chaque face principale d'une lentille ophtalmique selon l'invention sont déterminées de façon classique pour un faisceau lumineux incident arrivant sur la face principale depuis l'air, sans avoir traversé le substrat.

Enfin, une lentille ophtalmique selon l'invention présentant un paramètre $\Delta(\theta,\theta')$ tel que défini ci-dessus permet :

- de maximiser la réflexion de la lumière bleue phototoxique provenant du côté de la face principale avant, l'intensité de cette réflexion étant liée à la grandeur $R_\theta$(435 nm), et
- de minimiser la réflexion de la lumière bleue phototoxique provenant du côté de la face principale arrière, l'intensité de cette réflexion étant liée à la grandeur $R_{\theta'}$(435 nm).

Ainsi, la lentille ophtalmique selon l'invention, munie de son filtre, réduit la transmission globale de la lumière bleue phototoxique vers la rétine d'un porteur d'une telle lentille ophtalmique.

Le filtre proposé présentant une largeur à mi-hauteur plus importante qu'un filtre étroit, il s'avère moins épais qu'un tel filtre étroit et comprend moins de couches, et par conséquent est de fabrication plus aisée et moins coûteuse qu'un filtre étroit.

Par ailleurs, d'autres caractéristiques avantageuses et non limitatives de la lentille ophtalmique selon l'invention sont les suivantes :

- le filtre est formé sur la face principale avant de la lentille ophtalmique ;
- le paramètre $\Delta(\theta,\theta')$ est défini pour un angle d'incidence $\theta$ sur la face principale comportant ledit filtre tel que $\theta = 15°$ et pour un angle d'incidence $\theta'$ sur la face principale comportant ledit filtre tel que $\theta' = 45°$ ;
- le paramètre $\Delta_{spectral}$ est défini pour un angle d'incidence de 15° ;
- le paramètre $\Delta(\theta,\theta')$ est supérieur ou égal à 0,65, mieux supérieur ou égal à 0,7, mieux encore supérieur ou égal à 0,75, et de façon optimale supérieur ou égal à 0,8 ;
- le maximum de réflectivité est à une longueur d'onde inférieure ou égale à 410 nm, mieux inférieure ou égale à 400 nm, et encore mieux, inférieure ou égale à 390 nm ;
- le maximum de réflectivité est à une longueur d'onde supérieure ou égale à 350 nanomètres, de préférence dans la gamme de longueurs d'onde allant de 360 nm à 400 nm, mieux dans la gamme de longueurs d'onde allant de 370 nm à 390 nm ;
- la largeur à mi-hauteur est supérieure ou égale à 90 nanomètres, de préférence supérieure ou égale à 100 nanomètres ;
- la largeur à mi-hauteur est inférieure ou égale à 150 nanomètres, de préférence inférieure ou égale à 120 nanomètres, mieux inférieure ou égale à 110 nm.

Ainsi, la largeur à mi-hauteur est généralement comprise entre 80 nm et 150 nm, de préférence entre 90 nm et 120 nm, mieux entre 90 nm et 110 nm et mieux encore entre 100 nm et 110 nm.

Enfin, d'autres caractéristiques avantageuses et non limitatives de la lentille ophtalmique selon l'invention sont les suivantes :

- la valeur de la réflectivité au maximum de réflectivité de la face principale comportant le filtre, pour un angle d'incidence de 15°, est de préférence au moins 1,5 fois supérieure, mieux au moins 2 fois supérieure et de façon optimale au moins 2,5 fois supérieure à la valeur de la réflectivité de cette même face principale, pour le même angle d'incidence, et à la longueur d'onde de 435 nm ;
- le ratio $[\,R_{15°}(435\,nm) - R_{15°}(480\,nm)\,]\,/\,R_{15°}(435\,nm)$ où $R_{15°}(435\,nm)$ et $R_{15°}(480\,nm)$ représentent respectivement la réflectivité de la face principale de la lentille ophtalmique comportant ledit filtre à une longueur d'onde de 435 nm et à une longueur d'onde de 480 nm, pour un angle d'incidence sur cette face principale de 15°, est supérieur ou égal à 0,8, mieux supérieur ou égal à 0,85 et mieux encore supérieur ou égal à 0,9. Ce ratio caractérise l'excellente sélectivité du filtre équipant la lentille ophtalmique selon l'invention qui permet de protéger de la bande phototoxique sans perturbation de la bande chronobiologique ;
- le facteur moyen de réflexion lumineux ($R_v$) sur la face principale de la lentille ophtalmique comportant le filtre est inférieur ou égal à 2,5%, de préférence inférieur ou égal à 1,5% ;
- le facteur moyen de réflexion lumineux ($R_v$) sur chacune des faces principales de la lentille ophtalmique est inférieur ou égal à 2,5%, de préférence inférieur ou égal à 1,5% ;
- le facteur moyen de réflexion lumineux ($R_v$) sur la face principale de la lentille ophtalmique comportant le filtre est inférieur ou égal à 0,7% ;
- le filtre est formé sur la face principale avant de la lentille ophtalmique et le facteur moyen de réflexion (non pondéré) dans la gamme ultraviolette (UV) allant de 300 nm à 380 nm, pour un angle d'incidence sur cette face principale avant de 15°, est supérieur ou égal à 15%, mieux supérieur ou égal à 20% et mieux encore supérieur ou égal à 25% ;
- le filtre est un filtre interférentiel ;
- le filtre comporte un nombre de couches inférieur ou égal 11, préférentiellement de 2 à 10 couches, et encore plus préférentiellement de 4 à 9 couches et mieux encore de 4 à 7 couches ;
- le filtre présente une épaisseur totale inférieure ou égale à 700 nanomètres, de préférence inférieure ou égale à 600 nanomètres, et mieux encore inférieure ou égale à 550 nm, et de façon optimale de 200 nm à 400 nm ;
- la face principale arrière de la lentille ophtalmique comporte un revêtement anti-UV, c'est-à-dire réfléchissant peu les UV, préférentiellement un revêtement antireflet efficace dans les UV et dans le visible.

En outre, la lentille ophtalmique selon l'invention entre avantageusement dans la réalisation de paire de lunettes.

Ainsi, l'invention propose également une paire de lunettes comportant au moins une lentille ophtalmique selon l'invention.

Selon l'un de ses aspects, l'invention a pour objet l'utilisation d'une lentille ophtalmique selon l'invention pour accroître le contraste de vision d'un porteur. L'utilisation de lentilles selon l'invention permet ainsi d'améliorer le confort de vision du porteur, et notamment de reconnaître plus facilement les objets ou personnes observés au travers de ladite lentille ophtalmique. Cette utilisation trouvera son intérêt pour tout sujet, en particulier pour un sujet sain ne présentant pas de pathologies oculaires ou de prédisposition à une telle pathologie.

Par ailleurs, l'utilisation d'une lentille ophtalmique selon l'invention s'avère particulièrement intéressante pour un usage thérapeutique ou pour prévenir les maladies liées à la phototoxicité de la lumière bleue.

L'invention concerne donc également l'utilisation d'une lentille ophtalmique selon l'invention pour réduire les risques d'apparition d'une pathologie ophtalmique due à un processus dégénératif due à la photo-toxicité de la lumière bleue.

L'invention propose enfin l'utilisation d'une lentille ophtalmique selon l'invention pour protéger de la phototoxicité de la lumière bleue au moins une partie de l'œil d'un porteur, en particulier pour le protéger d'un processus dégénératif tel que la dégénérescence maculaire liée à l'âge (DMLA).

L'invention sera décrite plus en détail en référence aux dessins annexés, dans lesquels des lentilles ophtalmiques présentent un filtre selon l'invention sur leur face principale avant :

- les figures 1 à 3 présentent les courbes de réflectivité spectrale pour un angle d'incidence sur la face principale avant de 15°, de certaines lentilles ophtalmiques préparées dans les exemples 1 à 3 de la présente demande et de lentilles ophtalmiques revêtues d'un filtre ne satisfaisant pas les caractéristiques des filtres de l'invention, notamment la largeur à mi-hauteur (voir exemples comparatifs C1, C2, et C3).
- la figure 4 présente pour chacun des exemples précédents le facteur de transmission pondéré (en %) et la réflectivité arrière pondérée (en %), la pondération étant faite au moyen de la fonction de risque de la lumière bleue.
- la figure 5 représente les courbes de réflectivité spectrale entre 380 nm et 500 nm de la lentille ophtalmique de l'exemple 3 de la présente demande pour des angles d'incidence sur la face principale avant de 0° et 45°.

Comme cela est bien connu, la lentille ophtalmique selon l'invention comprend un substrat transparent en verre minéral ou organique. Ce substrat peut comprendre un ou plusieurs revêtements fonctionnels pour conférer à la lentille ophtalmique des propriétés optiques et/ou mécaniques particulières, tels que, par exemple, un revêtement anti-choc, un revêtement anti-abrasion, un revêtement anti-reflet, un revêtement anti-UV, un revêtement anti-statique, un revêtement polarisant, et un revêtement anti-salissure et/ou anti-buée. Tous ces revêtements sont bien connus dans la technique des lentilles ophtalmiques.

Le substrat de la lentille ophtalmique selon l'invention est de préférence en verre organique, par exemple une matière plastique thermoplastique ou thermodurcissable.

Parmi les matériaux thermoplastiques convenant pour les substrats, on peut citer les (co)polymères (méth) acryliques, en particulier le poly(méthacrylate de méthyle) (PMMA), les (co)polymères thio(méth)acryliques, le polyvinylbutyral (PVB), les polycarbonates (PC), les polyuréthanes (PU), les poly(thiouréthanes), les (co)polymères d'allylcarbonates de polyols, les copolymères thermoplastiques éthylène/acétate de vinyle, les polyesters tels que le poly(téréphtalate d'éthylène) (PET) ou le poly(téréphtalate de butylène) (PBT), les polyépisulfures, les polyépoxydes, les copolymères polycarbonates/polyesters, les copolymères de cyclo-oléfines tels que les copolymères éthylène/norbornène ou éthylène/cyclopentadiène et leurs combinaisons.

Par (co)polymère, on entend un copolymère ou un homopolymère. Par (méth)acrylate, on entend un acrylate ou un méthacrylate. Par polycarbonate (PC), on entend au sens de la présente invention aussi bien les homopolycarbonates que les copolycarbonates et les copolycarbonates séquencés.

Les substrats particulièrement recommandés sont les substrats obtenus par (co)polymérisation du bis allyl carbonate du diéthylèneglycol, vendu, par exemple, sous la dénomination commerciale CR-39® par la société PPG Industries (lentilles ORMA® ESSILOR), ou par polymérisation des monomères thio(méth)acryliques, tels que ceux décrits dans la demande de brevet français FR 2734827. Les substrats peuvent être obtenus par polymérisation de mélanges des monomères ci-dessus, ou peuvent encore comprendre des mélanges de ces polymères et (co)polymères.

D'autres substrats préférés sont les polycarbonates.

La lentille ophtalmique présente une face principale avant et une face principale arrière.

Par face principale arrière, on entend la face principale qui, lors de l'utilisation de la lentille ophtalmique, est la plus proche de l'œil de l'utilisateur. Il s'agit généralement d'une face concave. Inversement, par face principale avant, on entend la face principale qui, lors de l'utilisation de la lentille ophtalmique, est la plus éloignée de l'œil de l'utilisateur. Il s'agit généralement d'une face convexe.

Selon l'invention, l'une au moins des faces principales de la lentille ophtalmique comporte un filtre.

Comme indiqué précédemment, le substrat de la lentille ophtalmique peut comporter différents revêtements soit sur la face principale avant de la lentille ophtalmique, soit sur la face principale arrière de la lentille ophtalmique.

Un revêtement qui est "sur" le substrat ou qui a été déposé "sur" le substrat est défini comme un revêtement qui :

(i) est positionné au-dessus d'une face principale du substrat,
(ii) n'est pas nécessairement en contact avec le substrat, c'est-à-dire qu'un ou plusieurs revêtements intermédiaires peuvent être disposés entre le substrat et le revêtement en question, et

(iii) ne recouvre pas nécessairement complètement la face principale du substrat.

Lorsque "une couche A est localisée sous une couche B", on comprendra que la couche B est plus éloignée du substrat que la couche A.

Dans un mode de réalisation, le filtre est formé directement sur la face principale avant de la lentille ophtalmique.

Dans un autre mode préféré, il est déposé directement sur un revêtement anti-abrasion et/ou anti-rayures ayant lui-même été déposé sur la face principale avant de la lentille ophtalmique.

Avant le dépôt du filtre, il est courant de soumettre la surface dudit substrat, à un traitement d'activation physique ou chimique, destiné à augmenter l'adhésion du filtre sur la ou les faces principales.

Ce prétraitement est généralement conduit sous vide. Il peut s'agir d'un bombardement avec des espèces énergétiques, par exemple un faisceau d'ions (« *Ion Pre-Cleaning* » ou "IPC") ou un faisceau d'électrons, d'un traitement par décharge corona, par effluvage, d'un traitement UV, ou d'un traitement par plasma sous vide, généralement un plasma d'oxygène ou d'argon. Il peut également s'agir d'un traitement de surface acide ou basique et/ou par solvants (eau ou solvant organique).

Dans la présente demande, la réflectivité spectrale de la lentille ophtalmique, pour un angle d'incidence donné de la face comportant ledit filtre, représente la variation de la réflectivité (i.e. du facteur de réflexion) à cet angle d'incidence en fonction de la longueur d'onde. La courbe de réflectivité spectrale correspond à une représentation graphique de la réflectivité spectrale dans laquelle on trace la réflectivité spectrale (ordonnées) en fonction de la longueur d'onde (abscisses). Les courbes de réflectivité spectrales peuvent être mesurées au moyen d'un spectrophotomètre, par exemple un spectrophotomètre Perkin Elmer Lambda 850 équipé URA (Universal Reflectance Accessory).

Le facteur moyen de réflexion, noté $R_m$, est tel que défini dans la norme ISO 13666:1998, et mesuré conformément à la norme ISO 8980-4 (à un angle d'incidence inférieur à 17°, typiquement de 15°), c'est-à-dire qu'il s'agit de la moyenne (non pondérée) de la réflectivité spectrale sur l'ensemble du spectre lumineux allant de 400 nm à 700 nm.

De même, le facteur de réflexion lumineux, noté $R_v$, également appelé dans la présente demande facteur moyen de réflexion lumineux est tel que défini dans la norme ISO 13666:1998, et mesuré conformément à la norme ISO 8980-4 (à un angle d'incidence inférieur à 17°, typiquement de 15°), c'est-à-dire qu'il s'agit de la moyenne pondérée de la réflectivité spectrale sur l'ensemble du spectre lumineux visible compris entre 380 nm et 780 nm.

Par analogie, on définit un facteur moyen de réflexion dans le bleu entre 420 nm et 450 nm, noté $R_{m,B}$, qui correspond à la moyenne (non pondérée) de la réflectivité spectrale sur la gamme de longueurs d'onde allant de 420 nm à 450 nm.

Selon l'invention, ce facteur moyen de réflexion dans le bleu $R_{m,B}$ peut être mesuré pour un angle d'incidence sur la face principale comportant le filtre compris entre 0° (incidence normale) et 15°, préférentiellement à 15°.

Dans la présente demande, on notera également :

- $R_\theta$(435 nm) la valeur de la réflectivité de la face principale de la lentille ophtalmique comportant le filtre selon l'invention, cette valeur étant déterminée (par mesure ou par calcul) à une longueur d'onde de 435 nanomètres et pour un angle d'incidence $\theta$ sur la face principale comportant le filtre compris entre 0° et 15°, et
- $R_{\theta'}$(435 nm) la valeur de la réflectivité de la face principale de la lentille ophtalmique comportant le filtre selon l'invention, cette valeur étant déterminée (par mesure ou par calcul) à une longueur d'onde de 435 nanomètres et pour un angle d'incidence $\theta'$ sur la face principale comportant le filtre compris entre 30° et 45°.

On définira alors un paramètre $\Delta(\theta,\theta')$ par la relation suivante : $\Delta(\theta,\theta') = 1 - [\, R_{\theta'}(435\ nm)\, /\, R_\theta(435\ nm)\,]$. On verra dans la suite de la description comment ce paramètre $\Delta(\theta,\theta')$ permet d'évaluer l'efficacité d'une lentille ophtalmique à limiter la quantité de lumière bleue phototoxique arrivant sur la rétine d'un porteur, en tenant compte des contributions respectives de la lumière bleue provenant du côté de la face avant ou du côté de la face arrière de la lentille.

Conformément à l'invention, le filtre confère à la face principale de la lentille ophtalmique comprenant le filtre la propriété de présenter, pour un angle d'incidence sur cette face principale compris entre 0° et 15°, un facteur moyen de réflexion dans le bleu $R_{m,B}$ qui est supérieur ou égal à 5%.

Le filtre est ainsi spécialement conçu pour maximiser le facteur moyen de réflexion dans le bleu $R_{m,B}$. Ceci permet de maximiser la réjection de la lumière bleue phototoxique, dans la gamme de longueurs d'onde allant de 420 nm à 450 nm, arrivant de manière directe sur la face principale avant de la lentille. On considère ici que la majeure partie de la lumière directe qui provient de l'avant du porteur de la lentille ophtalmique et qui atteint la rétine de celui-ci présente une incidence faible sur la face principale avant, généralement comprise entre 0° et 15°.

Selon un mode de réalisation préféré de l'invention, le facteur moyen de réflexion dans le bleu $R_{m,B}$, pour un angle d'incidence sur la face principale de la lentille ophtalmique comportant le filtre compris entre 0° et 15°, de préférence 15°, est supérieur ou égal à 10%, mieux supérieur ou égal à 20%, et encore mieux supérieur ou égal à 30%, et de façon optimale supérieur ou égal à 50%.

Conformément à l'invention, le filtre confère également à la face principale comportant le filtre la propriété de présenter une courbe de réflectivité spectrale pour un angle d'incidence sur cette face principale compris entre 0° et 15°, de préférence 15°, qui a :

- un maximum de réflectivité à une longueur d'onde inférieure à 435 nanomètres, et
- une largeur à mi-hauteur (FWHM) supérieure ou égale à 80 nanomètres.

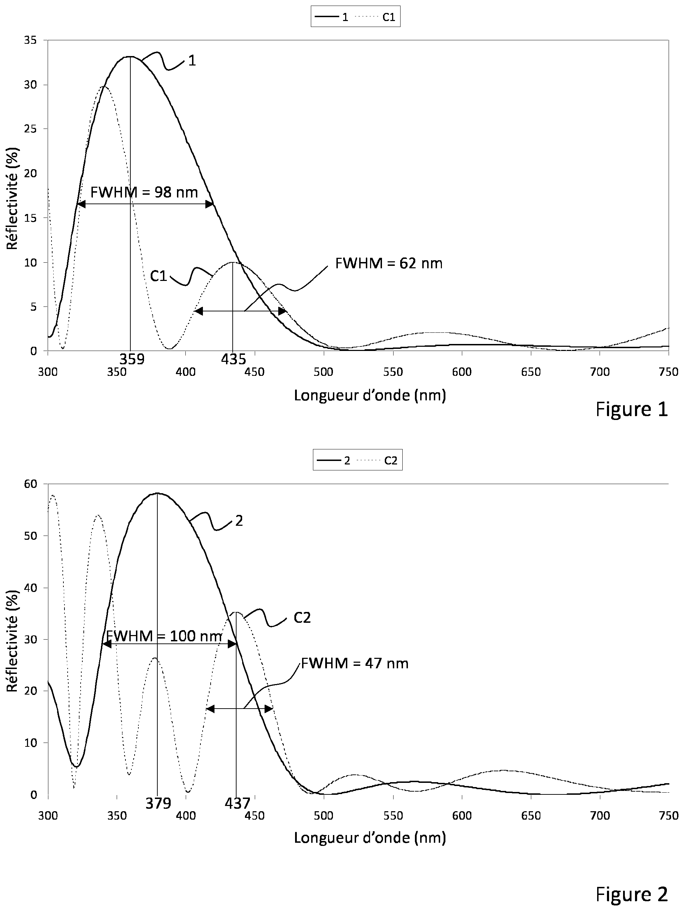

En effet, comme cela est visible sur les figures 1 à 3, les courbes de réflectivité spectrale de la face principale avant des lentilles ophtalmiques conformes à l'invention, présentent généralement, dans la gamme de longueurs d'onde allant de 380 nm à 500 nm, une forme de « cloche » que l'on peut caractériser par sa hauteur (maximum de réflectivité) et sa largeur à mi-hauteur (FWHM ou *« Full Width at Half Maximum »* en anglais).

Selon l'invention, le maximum de réflectivité est obtenu pour une longueur d'onde inférieure à 435 nm. Il est donc décalé par rapport à la longueur d'onde centrale (435 nm) de la bande de longueurs d'onde comprises entre 420 nm et 450 nm de la lumière bleue phototoxique.

De préférence, le maximum de réflectivité est à une longueur d'onde inférieure ou égale à 410 nm, mieux inférieure ou égale à 400 nm et encore mieux inférieure ou égale à 390 nm.

Dans un mode de réalisation préféré, ce décalage est limité de telle sorte que le maximum de réflectivité soit également à une longueur d'onde supérieure ou égale à 350 nm. De préférence, le maximum de réflectivité est à une longueur d'onde supérieure à 360 nm, mieux supérieure ou égale à 370 nm.

Selon l'invention, la largeur à mi-hauteur de la courbe de réflectivité spectrale considérée, pour un angle d'incidence sur la face principale comportant le filtre compris entre 0° et 15° est supérieure ou égale à 80 nm.

Un filtre dimensionné de telle sorte que la courbe de réflectivité spectrale pour un angle d'incidence sur la face principale comportant le filtre compris entre 0° et 15° ait une largeur à mi-hauteur (FWHM) supérieure ou égale à 80 nanomètres, sera désigné par la suite filtre large.

Dans un mode de réalisation préférée, la largeur à mi-hauteur est supérieure ou égale à 90 nanomètres, de préférence supérieure ou égale à 100 nanomètres.

De préférence également, la largeur à mi-hauteur est inférieure à 150 nanomètres, mieux inférieure à 120 nanomètres, mieux encore inférieure à 110 nm.

Toujours selon l'invention, le filtre confère enfin à la face principale de la lentille ophtalmique comportant le filtre la propriété de présenter un paramètre $\Delta(\theta,\theta')$, tel que défini précédemment, supérieur ou égal à 0,6.

Tel que défini précédemment, le paramètre $\Delta(\theta,\theta')$ dépend à la fois de la réflectivité à 435 nm pour un angle d'incidence $\theta$ sur la face principale compris entre 0° et 15°, notée $R_\theta(435\ nm)$ et à la fois de la réflectivité à 435 nm pour un angle d'incidence $\theta'$ sur la face principale compris entre 30° et 45°, notée $R_{\theta'}(435\ nm)$.

Dans le cas d'une lentille ophtalmique selon l'invention, placée devant l'œil d'un porteur, comme expliqué en introduction, on comprend que la quantité de lumière bleue phototoxique comprise dans la gamme de longueurs d'onde allant de 420 nm à 450 nm arrivant de manière directe sur la face principale avant de la lentille ophtalmique et parvenant à l'œil du porteur varie dans le sens inverse de la grandeur $R_\theta(435\ nm)$.

De la même manière, la quantité de lumière bleu phototoxique comprise dans la gamme de longueurs d'onde allant de 420 nm à 450 nm arrivant indirectement de l'arrière du porteur et réfléchie par la lentille ophtalmique varie dans le même sens que la grandeur $R_{\theta'}(435\ nm)$.

Ainsi, en choisissant un paramètre $\Delta(\theta,\theta')$ tel que $\Delta(\theta,\theta') \geq 0,6$, on obtient une lentille ophtalmique avec un filtre efficace et optimisé contre la lumière bleue phototoxique. En effet, le paramètre $\Delta(\theta,\theta')$ est d'autant plus élevé que :

(i) la valeur de la réflectivité $R_{\theta'}(435\ nm)$ est faible, c'est-à-dire que la quantité de lumière bleue phototoxique venant de l'arrière du porteur et réfléchie par la lentille ophtalmique en direction de la rétine du porteur est faible, et que

(ii) la valeur de la réflectivité $R_\theta(435\ nm)$ est élevée, c'est-à-dire que la quantité de lumière bleue phototoxique arrivant de manière directe sur la face principale avant de la lentille ophtalmique et réfléchie par celle-ci est élevée.

Dans un mode de réalisation préféré, le paramètre $\Delta(\theta,\theta')$ de la lentille ophtalmique munie d'un filtre large selon l'invention est supérieur ou égal à 0,7, mieux supérieur ou égal à 0,75, et mieux encore supérieur ou égal à 0,8.

De préférence, le paramètre $\Delta(\theta,\theta')$ est déterminé pour un angle d'incidence $\theta$ sensiblement égal à 15° et un angle d'incidence $\theta'$ sensiblement égal à 45°.

De préférence, le facteur moyen de transmission dans le bleu entre 465 nm et 495 nm de la lentille ophtalmique selon l'invention (pour un angle d'incidence sur la face principale avant compris entre 0° et 15°), qui correspond à la moyenne (non pondérée) de la transmitivité spectrale sur la gamme de longueurs d'onde allant de 465 nm à 495 nm, est supérieur ou égal à 80%, mieux supérieur ou égal à 85%, et encore mieux supérieur ou égal à 90%.

Ceci permet notamment de garantir que la majeure partie de la lumière bleue comprise entre 465 nm et 495 nm, qui serait responsable de la synchronisation de l'horloge biologique, soit transmise à l'œil d'un porteur équipé de cette lentille ophtalmique.

De manière préférée, le facteur de transmission de la lentille ophtalmique à 480 nm pour un angle d'incidence sur la face principale avant compris entre 0° et 15° est supérieur ou égal à 70%, mieux supérieur ou égal à 90%, et encore mieux supérieur ou égal à 95%.

Dans un mode de réalisation préféré de l'invention, le filtre que comporte la lentille est un filtre interférentiel. On entendra par là que le filtre comprend au moins une couche formée sur l'une des faces principales de la lentille ophtalmique munie du filtre interférentiel, cette couche présentant un indice de réfraction différent d'au moins 0,1 unité de l'indice de réfraction du substrat. Les propriétés optiques d'un tel filtre, comme par exemple la réflectivité, résultent des interférences provenant des réflexions multiples aux interfaces air / couche et substrat / couche.

Une couche du filtre est définie comme ayant une épaisseur déposée supérieure ou égale à 1 nm. Ainsi, toute couche ayant une épaisseur inférieure à 1 nm ne sera pas comptabilisée dans le nombre de couches du filtre. Une éventuelle sous-couche disposée entre le filtre et le substrat n'est pas non plus comptabilisée dans le nombre de couches du filtre interférentiel.

Sauf indication contraire, toutes les épaisseurs de couches divulguées dans la présente demande sont des épaisseurs physiques, et non des épaisseurs optiques.

Lorsque le filtre interférentiel de l'invention comprend au moins deux couches, il comprend alors un empilement d'au moins une couche de haut indice de réfraction, ou « couche haut indice » désignée couche HI, et d'au moins une couche de bas indice de réfraction, ou « couche bas indice » désignée couche BI.

Dans un mode de réalisation préféré, le filtre interférentiel comporte moins de 11 couches, de préférence un nombre de couches allant de 2 à 10 couches, mieux de 4 à 9 couches, de façon optimale de 4 à 7 couches. Il n'est pas nécessaire que les couches HI et BI soient alternées dans l'empilement du filtre interférentiel, bien qu'elles puissent l'être selon un mode de réalisation de l'invention. Deux couches HI (ou plus) peuvent être déposées l'une sur l'autre, tout comme deux couches BI (ou plus) peuvent être déposées l'une sur l'autre.

Dans la présente demande, une couche du filtre interférentiel est dite couche de haut indice de réfraction » lorsque son indice de réfraction est supérieur à 1,60, de préférence supérieur ou égal à 1,65, de préférence encore supérieur ou égal à 1,70, mieux supérieur ou égal à 1,80 et encore mieux supérieur ou égal à 1,90. De même, une couche du filtre interférentiel est dite couche de bas indice de réfraction lorsque son indice de réfraction est inférieur à 1,50, de préférence inférieur ou égal à 1,48, mieux inférieur ou égal à 1,47.

Sauf indication contraire, les indices de réfraction auxquels il est fait référence dans la présente demande sont exprimés à une température de 25°C et pour une longueur d'onde de référence égale à 550 nm.

La couche HI est une couche de haut indice de réfraction classique, bien connue dans la technique. Elle comprend généralement un ou plusieurs oxydes minéraux tels que, sans limitation : la zircone ($ZrO_2$), l'oxyde de titane ($TiO_2$), l'alumine ($Al_2O_3$), le pentoxyde de tantale ($Ta_2O_5$), l'oxyde de néodyme ($Nd_2O_5$), l'oxyde de praséodyme ($Pr_2O_3$), le titanate de praséodyme ($PrTiO_3$), l'oxyde de lanthane ($La_2O_3$), le pentoxyde de niobium ($Nb_2O_5$), ou l'oxyde d'yttrium ($Y_2O_3$). Éventuellement, les couches HI peuvent contenir également de la silice ou d'autres matériaux de bas indice de réfraction, pourvu que leur indice de réfraction soit supérieur à 1,60 comme indiqué ci-dessus. Les matériaux préférés sont $TiO_2$, $PrTiO_3$, $ZrO_2$, $Al_2O_3$, $Y_2O_3$ et leurs mélanges.

La couche BI est également couche de bas indice de réfraction classique bien connue et peut comprendre, sans limitation : de la silice ($SiO_2$), ou bien un mélange de silice et d'alumine, en particulier de la silice dopée avec de l'alumine, cette dernière contribuant à augmenter la résistance thermique du filtre interférentiel. La couche BI est de préférence une couche comprenant au moins 80% en masse de silice, mieux au moins 90 % en masse de silice, par rapport à la masse totale de la couche BI, et encore mieux consiste en une couche de silice.

Éventuellement, les couches bas indice peuvent contenir également des matériaux de haut indice de réfraction, pourvu que l'indice de réfraction de la couche résultante soit inférieur à 1,50.

Lorsqu'une couche BI comprenant un mélange de $SiO_2$ et d'$Al_2O_3$ est utilisée, elle comprend préférentiellement de 1 à 10%, mieux de 1 à 8% et encore mieux de 1 à 5% en masse d'$Al_2O_3$ par rapport à la masse totale de silice et d'alumine dans cette couche.

Par exemple, des couches de $SiO_2$ dopée avec 4% ou moins d'$Al_2O_3$ en masse, ou une couche de $SiO_2$ dopée avec 8% d'$Al_2O_3$ peuvent être employées. Des mélanges $SiO_2$ / $Al_2O_3$ disponibles dans le commerce peuvent être utilisés, tels que le LIMA® commercialisé par UMICORE MATERIALS AG (indice de réfraction compris entre 1,48 et 1,50), ou la substance L5® commercialisée par MERCK KGaA (indice de réfraction égale à 1,48 pour une longueur d'onde de 500 nm).

La couche externe du filtre interférentiel est généralement une couche bas indice, typiquement à base de silice, comprenant de préférence au moins 80% en masse de silice, mieux au moins 90% en masse de silice (par exemple une couche de silice dopée avec de l'alumine), par rapport à la masse totale de cette couche externe, et encore mieux consiste en une couche externe de silice.

Dans un mode de réalisation préféré, le filtre présente une épaisseur totale inférieure ou égale à 700 nanomètres, mieux inférieure ou égale à 600 nm. L'épaisseur totale du filtre est généralement supérieure à 200 nm, de préférence supérieure à 250 nm.

Dans les modes de réalisation particuliers de l'invention où le filtre est un filtre interférentiel comprenant 8 ou 9 couches, l'épaisseur totale de l'empilement est de préférence comprise entre 450 nm et 600 nm.

Dans les modes de réalisation particuliers de l'invention où le filtre est un filtre interférentiel comprenant 6 ou 7 couches, l'épaisseur totale de l'empilement est de préférence inférieure à 500 nm, mieux elle est comprise entre 300 nm et 500 nm.

Dans les modes de réalisation particuliers de l'invention où le filtre est un filtre interférentiel comprenant 4 ou 5 couches, l'épaisseur totale de l'empilement est de préférence inférieure à 300 nm, mieux elle est comprise entre 200 nm et 300 nm.

Généralement, les couches HI ont une épaisseur physique variant de 10 nm à 100 nm, mieux inférieure ou égale à 80 nm, et encore mieux inférieure ou égale à 70 nm, et les couches BI ont une épaisseur physique variant de 10 nm à 150 nm, mieux inférieure ou égale à 135 nm, et encore mieux inférieure ou égale à 120 nm.

La lentille ophtalmique de l'invention peut également être rendue antistatique, c'est-à-dire ne pas retenir et/ou développer une charge électrostatique appréciable, grâce à l'incorporation d'au moins une couche électriquement conductrice dans le filtre.

De préférence, il s'agit d'une couche supplémentaire d'un oxyde conducteur tel que l'oxyde d'indium, l'oxyde d'étain, l'ITO (Indium Tin Oxide). Cette couche présente une épaisseur généralement inférieure à 20 nm, préférentiellement entre 5 nm et 15 nm.

Elle est préférentiellement adjacente à une couche de haut indice telle qu'une couche d'oxyde de zirconium.

Préférentiellement, cette couche conductrice est disposée sous la dernière couche bas indice du filtre (c'est-à-dire la couche la plus proche de l'air), généralement à base de silice.

Selon un mode de réalisation de l'invention, le filtre est déposé sur une sous-couche. Il est considéré ici que cette sous-couche du filtre ne fait pas partie du filtre.

Par sous-couche du filtre, ou couche d'adhésion, on entend un revêtement d'épaisseur relativement importante, utilisé dans le but d'améliorer les propriétés mécaniques telles que la résistance à l'abrasion et/ou à la rayure du filtre et/ou de promouvoir son adhésion au substrat ou au revêtement sous-jacent.

Compte tenu de son épaisseur relativement importante, la sous-couche ne participe généralement pas à l'activité optique de filtrage du filtre, en particulier dans le cas où elle possède un indice de réfraction proche de celui du revêtement sous-jacent (qui est généralement le revêtement anti-abrasion et/ou anti-rayures) ou de celui du substrat de la lentille ophtalmique, lorsque la sous-couche est directement déposée sur le substrat de la lentille ophtalmique.

La sous-couche doit avoir une épaisseur suffisante pour promouvoir la résistance à l'abrasion du filtre, mais de préférence pas trop importante pour ne pas provoquer une absorption lumineuse qui, selon la nature de la sous-couche, pourrait réduire significativement le facteur de transmission visuelle $T_v$ tel que défini dans la norme ISO 13666 :1998, et mesuré conformément à la norme ISO 8980-3.

L'épaisseur de cette sous-couche est généralement inférieure à 300 nm, mieux inférieur à 200 nm, et est généralement supérieure à 90 nm, mieux supérieure à 100 nm.

La sous-couche comprend de préférence une couche à base de $SiO_2$, comprenant de préférence au moins 80% en masse de silice, mieux au moins 90% en masse de silice, par rapport à la masse totale de la sous-couche, et encore mieux consiste en une sous-couche de silice. L'épaisseur de cette sous-couche à base de silice est généralement inférieure à 300 nm, mieux inférieure à 200 nm, et est généralement supérieure à 90 nm, mieux supérieure à 100 nm.

Selon un autre mode de réalisation, cette sous-couche à base de $SiO_2$ est une sous-couche de silice dopée avec de l'alumine, dans des proportions telles que définies ci-dessus, de préférence consiste en une couche de silice dopée avec de l'alumine.

Selon un mode de réalisation particulier, la sous-couche consiste en une couche de $SiO_2$.

Il est préférable d'utiliser une sous-couche de type monocouche. Toutefois, la sous-couche peut être laminée (multicouches), en particulier lorsque la sous-couche et le revêtement sous-jacent (ou le substrat, si la sous-couche est déposée directement sur le substrat) présentent une différence d'indice de réfraction non négligeable. C'est notamment le cas lorsque le revêtement sous-jacent, qui est généralement un revêtement anti-abrasion et/ou anti-rayure, ou le substrat, possèdent un indice de réfraction élevé, terme par lequel on entend un indice de réfraction supérieur ou égal à 1,55, de préférence supérieur ou égal à 1,57.

Dans ce cas, la sous-couche peut comporter, outre une couche d'épaisseur comprise entre 90 nm et 300 nm, dite couche principale, de préférence au plus trois autres couches, mieux au plus deux autres couches, intercalées entre le substrat éventuellement revêtu et cette couche d'épaisseur comprise entre 90 nm et 300 nm, qui est généralement une couche à base de silice. Ces couches additionnelles sont de préférence de fines couches, dont la fonction est de limiter les réflexions multiples à l'interface sous-couche / revêtement sous-jacent ou à l'interface sous-couche / substrat, selon le cas.

Une sous-couche multicouche comprend préférentiellement, outre la couche principale, une couche d'indice de réfraction élevé et d'épaisseur inférieure ou égale à 80 nm, mieux inférieure ou égale à 50 nm et mieux encore inférieure ou égale à 30 nm. Cette couche d'indice de réfraction élevé est directement en contact avec le substrat d'indice de réfraction élevé ou le revêtement sous-jacent d'indice de réfraction élevé, selon le cas. Bien entendu, ce mode de réalisation peut être utilisé même si le substrat (ou le revêtement sous-jacent) possède un indice de réfraction inférieur à 1,55.

En alternative, la sous-couche comprend, outre la couche principale et la couche d'indice de réfraction élevé précitée, une couche de matériau d'indice de réfraction inférieur ou égal à 1,55, de préférence inférieur ou égal à 1,52, mieux inférieur ou égal à 1,50 à base de $SiO_2$ (c'est-à-dire comprenant de préférence au moins 80% en masse de silice) d'épaisseur inférieure ou égale à 80 nm, mieux inférieure ou égale à 50 nm et mieux encore inférieure ou égale à 30 nm, sur laquelle est déposée ladite couche d'indice de réfraction élevé. Typiquement, dans ce cas, la sous-couche comprend, déposées dans cet ordre sur le substrat éventuellement revêtu, une couche de 25 nm de $SiO_2$, une couche de 10 nm de $ZrO_2$ ou de $Ta_2O_5$ et la couche principale de la sous-couche.

Le filtre et l'éventuelle sous-couche sont préférentiellement déposés par dépôt sous vide selon l'une des techniques suivantes : i) par évaporation, éventuellement assistée par faisceau ionique ; ii) par pulvérisation par faisceau d'ions ; iii) par pulvérisation cathodique ; iv) par dépôt chimique en phase vapeur assisté par plasma. Ces différentes techniques sont décrites dans les ouvrages ″Thin Film Processes″ et ″Thin Film Processes II″ (Vossen & Kern Éditeurs, Academic Press, 1978 et 1991 respectivement). Une technique particulièrement recommandée est la technique d'évaporation sous vide.

De préférence, lorsque le filtre est un filtre interférentiel, le dépôt de chacune des couches de l'empilement du filtre et de l'éventuelle sous-couche est réalisé par évaporation sous vide.

Dans un mode de réalisation particulier de l'invention, la lentille ophtalmique présente un facteur moyen de réflexion lumineux $R_v$ sur la face principale de la lentille ophtalmique comportant le filtre qui est inférieur ou égal à 2,5%. De préférence, ce facteur moyen de réflexion lumineux $R_v$ est inférieur ou égal à 2%, mieux encore inférieur ou égal à 1,5%. Dans un mode particulièrement préféré, le facteur moyen de réflexion lumineux $R_v$ est inférieur ou égal à 0,7%, mieux inférieur ou égal à 0,6%.

Dans un mode de réalisation préféré, la lentille ophtalmique présente un facteur moyen de réflexion lumineux $R_v$ sur chacune des faces principales de la lentille ophtalmique qui est inférieur ou égal à 2,5%. Mieux, ce facteur moyen de réflexion lumineux $R_v$ est inférieur ou égal à 0,7%.

Selon un mode de réalisation préféré de l'invention, la face principale revêtue par le filtre selon l'invention est la face principale avant de la lentille ophtalmique de l'invention et la face principale arrière est revêtue d'un revêtement antireflet conventionnel ou bien de préférence d'un revêtement antireflet efficace dans l'UV, c'est-à-dire réfléchissant peu les UV, tel que ceux décrits par exemple dans le document PCT/EP2011/072386.

Le facteur moyen de réflexion dans l'UV $R_{UV}$ sur la face principale arrière de la lentille ophtalmique, pour des longueurs d'onde comprises entre 280 nm et 380 nm, pondéré par la fonction W($\lambda$) définie dans la norme ISO 13666:1998, est inférieur ou égal à 7%, mieux inférieur ou égal à 6%, et mieux encore inférieur ou égal à 5%, pour un angle d'incidence de 30° et pour un angle d'incidence de 45°. Le facteur moyen de réflexion dans l'UV $R_{UV}$ est défini par la relation :

$$R_{UV} = \frac{\int\limits_{280}^{380} W(\lambda).R(\lambda).d\lambda}{\int\limits_{280}^{380} W(\lambda).d\lambda}$$

où R($\lambda$) désigne la réflectivité spectrale sur la face principale arrière de la lentille ophtalmique à la longueur d'onde considérée, et W($\lambda$) désigne une fonction de pondération égale au produit de l'éclairement énergétique spectral solaire Es($\lambda$) par la fonction spectrale relative d'efficacité S($\lambda$).

La fonction spectrale W($\lambda$), qui permet de calculer les facteurs de transmission des rayonnements UV est définie dans la norme ISO 13666:1998.

Le revêtement antireflet efficace dans l'UV comprend de préférence un empilement d'au moins une couche de haut indice de réfraction et d'au moins une couche de bas indice de réfraction.

Dans un autre mode de réalisation de l'invention, les deux faces principales avant et arrière comportent chacune un filtre contre la lumière bleue phototoxique. Les deux filtres ainsi formés, l'un sur la face principale avant et l'autre sur la face principale arrière, peuvent alors être identiques ou différents.

Le filtre selon l'invention peut être déposé directement sur un substrat nu. Dans certaines applications, il est préférable que la face principale de la lentille ophtalmique comportant le filtre soit revêtue d'un ou plusieurs revêtements fonctionnels préalablement à la formation du filtre sur cette face principale. Ces revêtements fonctionnels, classiquement utilisés en optique, peuvent être, sans limitation : une couche de primaire anti-choc, un revêtement anti-abrasion et/ou anti-rayures, un revêtement polariseur, un revêtement coloré.

Généralement, la face principale avant et/ou arrière du substrat sur laquelle sera formé un filtre est revêtue d'une couche de primaire anti-choc, d'un revêtement anti-abrasion et/ou anti-rayures.

Le filtre est de préférence déposé sur un revêtement anti-abrasion et/ou anti-rayures. Le revêtement anti-abrasion et/ou anti-rayures peut être toute couche classiquement utilisée comme revêtement anti-abrasion et/ou anti-rayure dans le domaine des lentilles ophtalmiques. De tels revêtements sont décrits, entre autres, dans le document EP 0614957.

La lentille ophtalmique selon l'invention peut également comporter des revêtements formés sur le filtre et capables de modifier ses propriétés de surface, tels que des revêtements hydrophobes et/ou oléophobes (« *top coat* » anti-salissure) et/ou des revêtements anti-buée. De tels revêtements sont décrits, entre autres, dans le document US 7678464. Ces revêtements sont de préférence déposés sur la couche externe du filtre. Leur épaisseur est en général inférieure ou égale à 10 nm, de préférence de 1 nm à 10 nm, mieux de 1 nm à 5 nm.

Typiquement, une lentille ophtalmique selon l'invention comprend un substrat successivement revêtu sur sa face principale avant d'une couche de primaire anti-choc, d'une couche anti-abrasion et/ou anti-rayures, d'un filtre selon l'invention, et d'un revêtement hydrophobe et/ou oléophobe.

La lentille ophtalmique selon l'invention est de préférence une lentille ophtalmique pour lunettes, ou une ébauche de lentille ophtalmique. L'invention concerne ainsi également une paire de lunettes comportant au moins une telle lentille ophtalmique.

La lentille ophtalmique peut être une lentille polarisée, ou une lentille solaire, teintée, avec ou sans correction.

La face principale arrière du substrat de l'article d'optique peut être revêtue successivement d'une couche de primaire anti-choc, d'une couche anti-abrasion et/ou anti-rayures, d'un revêtement anti-reflet qui peut être, ou non, un revêtement antireflet anti-UV, et d'un revêtement hydrophobe et/ou oléophobe.

Elle est particulièrement avantageuse pour protéger de la phototoxicité de la lumière bleue l'œil d'un porteur souffrant d'une détérioration d'un œil, en particulier due à un processus dégénératif tel que la dégénérescence maculaire liée à l'âge.

Une lentille ophtalmique telle que décrite ci-dessus présente également l'avantage de conférer un meilleur contraste visuel au porteur.

Les exemples suivants illustrent l'invention de façon plus détaillée mais non limitative.

EXEMPLES

1. Procédures générales et modes opératoires

Les filtres selon l'invention sont déposés sur des verres ORMA® revêtus d'un revêtement anti-abrasion tel que décrit dans l'exemple 3 du brevet EP614957.

Le dispositif d'évaporation et les conditions de dépôt des couches de $SiO_2$ et de $ZrO_2$ (vitesse d'évaporation, pression) sont telles que décrites dans la demande de brevet WO 2008107325.

2. Calcul des courbes

Les courbes de réflectivité spectrales des filtres selon l'invention ont été modélisées à partir du logiciel *Essential Mac Leod* (version 9.4) de Thin Film Center.

Les caractéristiques des filtres et leurs propriétés figurent au point 3 ci-dessous.

Les lentilles ophtalmiques équipées des filtres des exemples 1 et 2 ont effectivement été réalisées et les courbes de réflectivités spectrales mesurées.

Il a été vérifié que les courbes obtenues correspondaient à celles modélisées.

3. Empilements des filtres et propriétés. Courbes de réflectivité spectrale. Résultats

Les caractéristiques structurelles et les performances optiques des lentilles ophtalmiques obtenues selon les exemples 1 à 3 sont détaillées dans le tableau ci-dessous (voir page suivante).

Les courbes de réflectivité spectrale à un angle d'incidence sur la face principale avant de 15° et pour des longueurs d'onde allant de 280 nm à 780 nm des exemples 1 à 3 ci-dessous sont représentées sur les figures 1 à 3. Sont également représentées sur ces figures, les courbes de réflectivité spectrale des exemples comparatifs C1, C2, et C3 (voir ci-dessous).

Les valeurs des facteurs moyens de réflexion sont celles de la face principale avant. Les facteurs $R_{m,B}$ et $R_V$ sont indiqués pour un angle d'incidence de 15°.

Dans le tableau suivant, le paramètre Δ **spectral @ 15°** est défini par la relation : Δ **spectral @ 15°** = [ $R_{15°}$(435 nm) - $R_{15°}$(480 nm)] / $R_{15°}$(435 nm) où $R_{15°}$( 435 nm) et $R_{15°}$(480 nm) représentent respectivement la réflectivité de la face principale avant à 435 nm et à 480 nm, pour un angle d'incidence de 15° sur la face principale avant.

On constate que les lentilles ophtalmiques selon l'invention possèdent de très bonnes propriétés de réflexion de la lumière bleue phototoxique ($R_{m,B}$ > 10%), sans que cela ne nuise aux performances anti-reflet dans le domaine visible ($R_v$ < 2,5% pour un angle d'incidence de 15°).

Les lentilles ophtalmiques obtenues selon les exemples 1 à 3 présentent en outre d'excellentes propriétés de transparence et une bonne neutralité colorimétrique, une bonne résistance à l'abrasion et aux rayures, et une bonne résistance à un traitement au trempé dans l'eau chaude suivi d'une sollicitation mécanique de surface. L'adhérence des revêtements au substrat est également très satisfaisante.

| Ex. 1 : filtre large à 4 couches sur la face principale avant | | Ex. 2 : filtre large à 6 couches sur la face principale avant | | Ex. 3 : filtre large à 8 couches sur la face principale avant | |
|---|---|---|---|---|---|
| Substrat + hard coat | | Substrat + hard coat | | Substrat + hard coat | |
| ZrO2 | 34 nm | ZrO2 | 44 nm | ZrO2 | 47 nm |
| SiO2 | 35 nm | SiO2 | 45 nm | SiO2 | 50 nm |
| ZrO2 | 73 nm | ZrO2 | 68 nm | ZrO2 | 54 nm |
| SiO2 | 110 nm | SiO2 | 32 nm | SiO2 | 70 nm |
| Air | | ZrO2 | 66 nm | ZrO2 | 45 nm |
| | | SiO2 | 124 nm | SiO2 | 62 nm |
| | | Air | | ZrO2 | 53 nm |
| | | | | SiO2 | 134 nm |
| | | | | Air | |
| Épaisseur totale | 252 nm | Épaisseur totale | 379 nm | Épaisseur totale | 515 nm |
| Rm,B @ 15° (420 - 450nm) | 11,8% | Rm,B @ 15° (420 - 450nm) | 30,6% | Rm,B @ 15° (420 - 450nm) | 51,5% |
| Max. Réflectivité | 359 nm | Max. Réflectivité | 379 nm | Max. Réflectivité | 384 nm |
| Largeur à mi-hauteur | 98 nm | largeur à mi-hauteur | 100 nm | largeur à mi-hauteur | 105 nm |
| Δ(θ=15°,θ'=45°) @ 435 nm | 0,72 | Δ(θ=15°,θ'=45°) @ 435 nm | 0,75 | Δ(θ=15°,θ'=45°) @ 435 nm | 0,80 |
| Rm @ 15° (465 - 495 nm) | 2,3% | Rm @ 15° (465 - 495 nm) | 4,5% | Rm @ 15° (465 - 495 nm) | 7,3% |
| Δ spectral @ 15° 435 nm/480 nm | 0,85 | Δ spectral @ 15° 435 nm/480 nm | 0,92 | Δ spectral @ 15° 435 nm/480 nm | 0,95 |
| Rv @ 15° (380 - 780 nm) | 0,5% | Rv @ 15° (380 - 780 nm) | 1,9% | Rv @ 15° (380 - 780 nm) | 2,0% |

Exemples comparatifs C1, C2, et C3

Les performances « anti-lumière bleue » de trois lentilles ophtalmiques équipées d'un filtre ne satisfaisant pas les caractéristiques des filtres de l'invention, notamment la largeur à mi-hauteur, sont consignées dans les tableaux ci-dessous (voir page suivante).

On constate que les lentilles ophtalmiques équipées de filtres selon l'invention présentent un meilleur facteur de réflexion lumineux $R_v$ que les lentilles comparatives, en particulier les filtres possédant un nombre restreint de couches (nombre de couches inférieures ou égales à 7, préférentiellement inférieures ou égales à 6 couches).

On constate que toutes les lentilles ophtalmiques des exemples comparatifs C1, C2, et C3 qui sont munies de filtres ne satisfaisant pas les caractéristiques des filtres de l'invention, notamment la largeur à mi-hauteur, présentent un facteur moyen de réflexion dans le bleu $R_{m,B}$ supérieur ou égal à 5%.

En revanche, comme cela est visible sur les figures 1 à 3, aucun exemple comparatif de ces lentilles ophtalmiques ne présente :

- un maximum de réflectivité à une longueur d'onde inférieure à 435 nm, et

- une largeur à mi-hauteur supérieure ou égale à 80 nm.

| **Ex. comparatif C1 : filtre « étroit » à 4 couches sur la face principale avant** | | **Ex. comparatif C2 : filtre « étroit » à 6 couches sur la face principale avant** | | **Ex. comparatif C3 : filtre « étroit » à 8 couches sur la face principale avant** | |
|---|---|---|---|---|---|
| **Substrat + hard coat** | | **Substrat + hard coat** | | **Substrat + hard coat** | |
| **ZrO2** | 45 nm | **ZrO2** | 34 nm | **ZrO2** | 163 nm |
| **SiO2** | 15 nm | **SiO2** | 43 nm | **SiO2** | 220 nm |
| **ZrO2** | 235 nm | **ZrO2** | 315 nm | **ZrO2** | 182 nm |
| **SiO2** | 98 nm | **SiO2** | 25 nm | **SiO2** | 51 nm |
| **Air** | | **ZrO2** | 196 nm | **ZrO2** | 47 nm |
| | | **SiO2** | 115 nm | **SiO2** | 26 nm |
| | | **Air** | | **ZrO2** | 121 nm |
| | | | | **SiO2** | 107 nm |
| | | | | **Air** | |
| **Épaisseur totale** | 393 nm | **Épaisseur totale** | 728 nm | **Épaisseur totale** | 917 nm |
| **Rm,B @ 15° (420 - 450nm)** | 8,5% | **Rm,B @ 15° (420 - 450nm)** | 35,0% | **Rm,B @ 15° (420 - 450nm)** | 55,0% |
| **Max. Réflectivité** | 435 nm | **Max. Réflectivité** | 437 nm | **Max. Réflectivité** | 436 nm |
| **largeur à mi-hauteur** | 62 nm | **largeur à mi-hauteur** | 47 nm | **largeur à mi-hauteur** | 54 nm |
| $\Delta(\theta=0°,\theta'=45°)$ **@ 435 nm** | 0,40 | $\Delta(\theta=0°,\theta'=45°)$ **@ 435 nm** | 0,55 | $\Delta(\theta=0°,\theta'=45°)$ **@ 435 nm** | 0,57 |
| **Rm @ 15° (465 - 495 nm)** | 3,5% | **Rm @ 15° (465 - 495 nm)** | 7,5% | **Rm @ 15° (465 - 495 nm)** | 13,8% |
| $\Delta$ **spectral @ 15° 435 nm/480 nm** | 0,40 | $\Delta$ **spectral @ 15° 435 nm/480 nm** | 0,90 | $\Delta$ **spectral @ 15° 435 nm/480 nm** | 0,93 |
| **Rv @ 15° (380 - 780 nm)** | 1,4% | **Rv @ 15° (380 - 780 nm)** | 2,6% | **Rv @ 15° (380 - 780 nm)** | 2,1% |

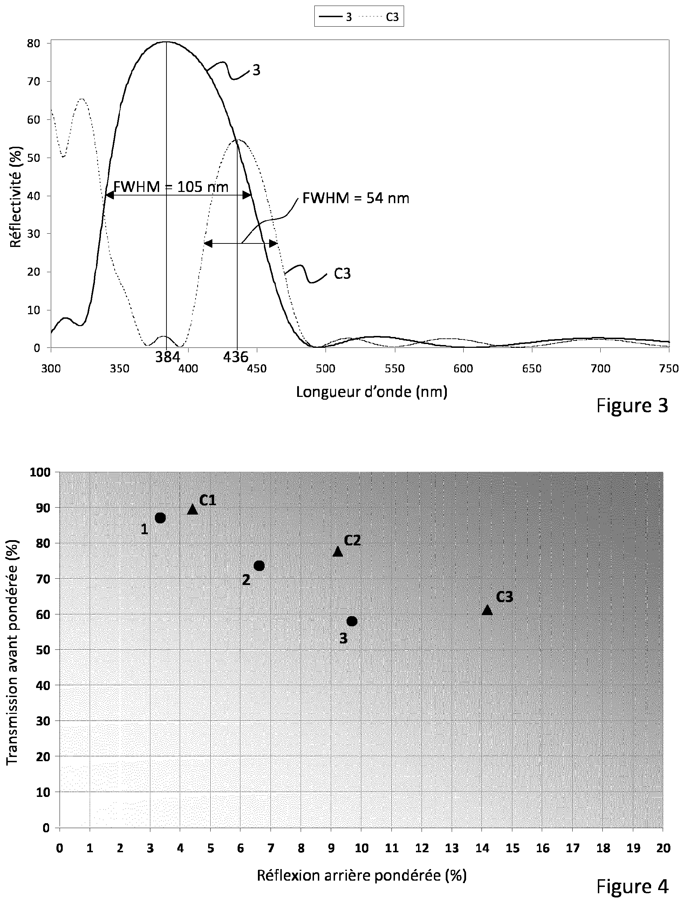

Par ailleurs, on comprendra à la lumière de la figure 4 l'efficacité des exemples 1 à 3 de lentilles ophtalmiques selon l'invention par rapport aux exemples comparatifs C1, C2, et C3 décrits ci-dessus.

La réflexion arrière $BR(\lambda)$ et la transmission $T(\lambda)$ de l'ensemble du système optique (avec le filtre bleu correspondant aux exemples 1, 2 et 3 et aux exemples comparatifs C1, C2 et C3, en face avant d'une lentille de type biplan en verre ORMA®, portant un antireflet Crizal Forte® UV en face arrière ($R_v$ = 0,59%, $R_{UV}$ = 3,1% pour une incidence de 45°), ont été déterminées à l'aide du logiciel *Essential Mac Leod* pour chacun des filtres étudiés.

Le calcul tient compte de l'ensemble des multiples réflexions se produisant au sein de la lentille ophtalmique.

Pour évaluer le risque associé à la lumière bleue, ces courbes de transmission et de réflexion sont pondérées à l'aide de la fonction spectrale $W_B(\lambda)$ issue de la norme internationale ISO 8980-3. Cette fonction résulte du produit de la fonction de risque de la lumière bleue (« *blue-light hazard function* » en anglais) $B(\lambda)$ et de la fonction de distribution spectrale du soleil (« *spectral distribution of solar radiation* » en anglais) $E_S(\lambda)$ intégrées sur la gamme de longueurs d'onde allant de 380 nm à 500 nm (voir Tableau 1 page suivante).

Sur la figure 4, sont représentés :

- en abscisses : la valeur de la réflexion arrière pondérée par la fonction de pondération du risque de la lumière bleue, pour un angle d'incidence sur la face principale arrière de 45°.

$$BR_B = \frac{\int_{380}^{500} W_B(\lambda).BR(\lambda).d\lambda}{\int_{380}^{500} W_B(\lambda).d\lambda}$$

où *BR(λ)* est le facteur spectral de réflexion arrière du verre (« *back reflectance* » en anglais), et

- en ordonnées : la valeur de la transmission pondérée par la fonction de pondération du risque de la lumière bleue. Cette valeur de transmission représente la part (en %) de lumière directe transmise dans la bande bleu-violet (de 380 nm à 500 nm) par cette lentille ophtalmique pour un angle d'incidence sur la face principale avant de 0°.

$$T_B = \frac{\int_{380}^{500} W_B(\lambda).T(\lambda).d\lambda}{\int_{380}^{500} W_B(\lambda).d\lambda}$$

où *T(λ)* est le facteur spectral de transmission du verre.

La grandeur $W_B(\lambda)$ représente la fonction de pondération qui est égale au produit de l'irradiation solaire spectrale *Es(λ)* et de la fonction de risque de la lumière bleue *B(λ)* (voir tableau 1).

On constate sur la figure 4 que les exemples 1 à 3 de lentilles ophtalmiques selon l'invention présentent non seulement une transmission plus faible, mais aussi une réflexion arrière plus faible, que les exemples comparatifs C1, C2, et C3, à nombre de couches égal.

Ainsi, les lentilles ophtalmiques selon l'invention permettent de prévenir un processus dégénératif de l'œil d'un porteur dû à la phototoxicité de la lumière bleue, tel que la dégénérescence maculaire liée à l'âge.

*Fonctions spectrales pour le calcul des valeurs de transmission ou réflexion de la lumière dans la bande 380-500 nm :*

**Tableau 1 :** Données numériques permettant de calculer la fonction de pondération $W_B(\lambda)$.

| Longueur d'onde λ (nm) | Irradiation solaire spectrale Es(λ) (mW/m².nm) | Fonction de risque de la lumière bleue B(λ) | Fonction de pondération $W_B(\lambda) = Es(\lambda).B(\lambda)$ |
|---|---|---|---|
| 380 | 336 | 0.006 | 2 |
| 385 | 365 | 0,012 | 4 |
| 390 | 397 | 0,025 | 10 |
| 395 | 432 | 0,05 | 22 |
| 400 | 470 | 0,10 | 47 |
| 405 | 562 | 0,20 | 112 |
| 410 | 672 | 0,40 | 269 |
| 415 | 705 | 0,80 | 564 |
| 420 | 733 | 0,90 | 660 |
| 425 | 760 | 0,95 | 722 |
| 430 | 787 | 0,98 | 771 |
| 435 | 849 | 1,00 | 849 |

(suite)

| Longueur d'onde $\lambda$ (nm) | Irradiation solaire spectrale Es($\lambda$) (mW/m$^2$.nm) | Fonction de risque de la lumière bleue B($\lambda$) | Fonction de pondération $W_B(\lambda)$ = Es($\lambda$).B($\lambda$) |
|---|---|---|---|
| 440 | 911 | 1,00 | 911 |
| 445 | 959 | 0,97 | 930 |
| 450 | 1006 | 0,94 | 946 |
| 455 | 1037 | 0,90 | 933 |
| 460 | 1080 | 0,80 | 864 |
| 465 | 1109 | 0,70 | 776 |
| 470 | 1138 | 0,62 | 706 |
| 475 | 1161 | 0,55 | 639 |
| 480 | 1183 | 0,45 | 532 |
| 485 | 1197 | 0,40 | 479 |
| 490 | 1210 | 0,22 | 266 |
| 495 | 1213 | 0,16 | 194 |
| 500 | 1215 | 0,10 | 122 |

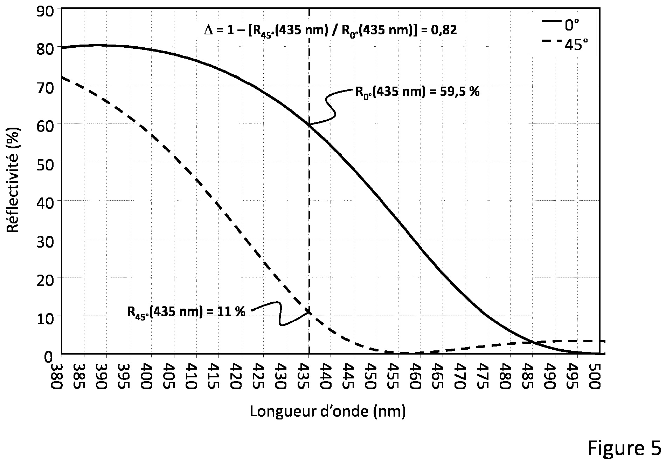

La figure 5 représente les courbes de réflectivité spectrale entre 380 nm et 500 nm de la lentille ophtalmique de l'exemple 3 pour des angles d'incidence sur la face principale avant de 0° et de 45°.

On remarque sur cette figure que la courbe de réflectivité spectrale à 45° est décalée vers les courtes longueurs d'onde (i.e. vers le bleu profond et l'UV) par rapport à la courbe de réflectivité spectrale à 0°. Ceci est l'illustration de la forte sélectivité angulaire du filtre large de l'exemple 3.

Ce décalage entraîne alors que la valeur de la réflectivité spectrale à 435nm pour un angle d'incidence de 45°, notée ici $R_{45°}$(435 nm) est faible, ici égale à 11%, et bien inférieure à la valeur de la réflectivité spectrale à 435nm pour un angle d'incidence de 0°, notée ici $R_{0°}$(435 nm), égale à 59,5%.

On comprend donc que la valeur du paramètre $\Delta(\theta,\theta')$ est ici élevée, égale à 0,82. Ceci est vrai pour toutes les lentilles ophtalmiques, qui sont munies d'au moins un filtre large selon l'invention.

Au contraire, et comme cela est visible sur les tableaux des exemples comparatifs, une lentille ophtalmique munie d'un filtre ne satisfaisant pas les caractéristiques des filtres de l'invention, notamment la largeur à mi-hauteur, ne présente pas de paramètre $\Delta(\theta,\theta')$ suffisamment élevé pour être efficace contre la lumière bleue phototoxique.

## Revendications

1. Lentille ophtalmique présentant une face principale avant et une face principale arrière, l'une au moins des faces principales comportant un filtre qui confère à la face principale comportant ledit filtre les propriétés suivantes :

   - un facteur moyen de réflexion dans le bleu ($R_{m,B}$) sur une gamme de longueurs d'onde allant de 420 nanomètres à 450 nanomètres qui est supérieur ou égal à 5 %, pour un angle d'incidence compris entre 0° et 15°,
   - une courbe de réflectivité spectrale pour un angle d'incidence compris entre 0° et 15°, cette courbe de réflectivité ayant :

      - un maximum de réflectivité à une longueur d'onde inférieure à 435 nanomètres, et
      - une largeur à mi-hauteur (FWHM) supérieure ou égale à 80 nanomètres, **caractérisée en ce que**

   - la valeur de la réflectivité au maximum de réflectivité de la face principale comportant ledit filtre, pour un angle d'incidence de 15°, est au moins 1,5 fois supérieure à la valeur de la réflectivité de cette même face principale, à la longueur d'onde de 435 nm et pour un angle d'incidence de 15°, et
   - pour un angle d'incidence $\theta$ compris entre 0° et 15° et pour un angle d'incidence $\theta'$ compris entre 30° et 45°, un paramètre $\Delta(\theta,\theta')$ défini par la relation $\Delta(\theta,\theta') = 1 - [ R_{\theta'}(435\text{ nm}) / R_{\theta}(435\text{ nm}) ]$, tel que ce paramètre $\Delta(\theta,\theta')$ soit supérieur ou égal à 0,6, où

- $R_\theta$(435 nm) représente la valeur de la réflectivité de la face principale comportant ledit filtre à la longueur d'onde de 435 nanomètres pour l'angle d'incidence $\theta$, et
- $R_{\theta'}$(435 nm) représente la valeur de la réflectivité de la face principale comportant ledit filtre à la longueur d'onde de 435 nanomètres pour l'angle d'incidence $\theta'$.

2. Lentille ophtalmique selon la revendication 1, dans laquelle le paramètre $\Delta(\theta,\theta')$ est défini pour un angle d'incidence $\theta$ = 15° et un angle d'incidence $\theta'$ = 45°.

3. Lentille ophtalmique selon la revendication 1 ou 2, dans laquelle le facteur moyen de réflexion dans le bleu ($R_{m,B}$) est supérieur ou égal à 10 %, mieux supérieur ou égal à 20 %, et mieux encore, supérieur ou égal à 30 %, pour un angle d'incidence compris entre 0° et 15°.

4. Lentille ophtalmique selon l'une des revendications 1 3, dans laquelle le maximum de réflectivité est à une longueur d'onde inférieure ou égale à 410 nm, mieux inférieure ou égale à 400 nm, et encore mieux inférieure ou égale à 390 nm.

5. Lentille ophtalmique selon l'une des revendications 1 à 4, dans laquelle le maximum de réflectivité est à une longueur d'onde supérieure ou égale à 350 nm, mieux supérieure ou égale à 360 nm.

6. Lentille ophtalmique selon l'une des revendications 1 à 5, dans laquelle la largeur à mi-hauteur est supérieure ou égale à 90 nanomètres, de préférence supérieure ou égale à 100 nanomètres.

7. Lentille ophtalmique selon l'une des revendications 1 à 6, dans laquelle la largeur à mi-hauteur est inférieure ou égale à 150 nanomètres, de préférence inférieure ou égale à 120 nanomètres, mieux inférieure ou égale à 110 nm.

8. Lentille ophtalmique selon l'une des revendications 1 à 7, dans laquelle le facteur moyen de réflexion lumineux ($R_v$) sur la face principale de la lentille ophtalmique comportant le filtre est inférieur ou égal à 2,5%, préférentiellement inférieur ou égal à 1,5%.

9. Lentille ophtalmique selon l'une des revendications 1 à 7, dans laquelle le facteur moyen de réflexion lumineux ($R_v$) sur chacune des faces principales de la lentille ophtalmique est inférieur ou égal à 2,5%, préférentiellement inférieur ou égal à 1,5%.

10. Lentille ophtalmique selon l'une des revendications 1 à 9, dans laquelle le filtre est un filtre interférentiel.

11. Lentille ophtalmique selon la revendication 10, dans laquelle le filtre comporte un nombre de couches inférieur ou égal à 11, préférentiellement de 2 à 10 couches, et encore plus préférentiellement de 4 à 9 couches.

12. Lentille ophtalmique selon l'une des revendications 1 à 11, dans laquelle le filtre présente une épaisseur totale inférieure ou égale à 700 nanomètres, de préférence inférieure ou égale à 600 nanomètres.

13. Lentille ophtalmique selon l'une des revendications 1 à 12, **caractérisée en ce que** le ratio [ $R_{15°}$(435 nm) - $R_{15°}$(480 nm) ] / $R_{15°}$(435 nm) est supérieur ou égal à 0,8, où $R_{15°}$(435 nm) et $R_{15°}$(480 nm) représentent respectivement la réflectivité de la face principale comportant ledit filtre à 435 nm et à 480 nm, pour un angle d'incidence de 15° sur cette face principale.

14. Lentille ophtalmique selon l'une des revendications 1 à 13, **caractérisé en ce que** la valeur de la réflectivité au maximum de réflectivité de la face principale comportant ledit filtre, pour un angle d'incidence de 15°, est au moins 2 fois supérieure à la valeur de la réflectivité de cette même face principale, à la longueur d'onde de 435 nm et pour un angle d'incidence de 15°.

15. Lentille ophtalmique selon l'une des revendications 1 à 14, dans laquelle le filtre est formé sur la face principale avant de la lentille ophtalmique.

16. Lentille ophtalmique selon la revendication 15, dans laquelle la face principale arrière de la lentille ophtalmique comporte un revêtement antireflet efficace dans l'UV ayant un facteur moyen de réflexion dans l'UV $R_{UV}$ sur la face principale arrière de la lentille ophtalmique, pour des longueurs d'onde comprises entre 280 nm et 380 nm, pondéré par la fonction $W(\lambda)$ définie dans la norme ISO 13666:1998, inférieur ou égal à 7%, pour un angle d'incidence de 30° et pour un angle d'incidence de 45°, le facteur moyen de réflexion dans l'UV $R_{UV}$ étant défini par la relation :

$$R_{UV} = \frac{\int\limits_{280}^{380} W(\lambda).R(\lambda).d\lambda}{\int\limits_{280}^{380} W(\lambda).d\lambda}$$

où $R(\lambda)$ désigne la réflectivité spectrale sur la face principale arrière de la lentille ophtalmique à la longueur d'onde considérée, et $W(\lambda)$ désigne une fonction de pondération égale au produit de l'éclairement énergétique spectral solaire $Es(\lambda)$ par la fonction spectrale relative d'efficacité $S(\lambda)$.

17. Lentille ophtalmique présentant une face principale avant et une face principale arrière, l'une au moins des faces principales comportant un filtre qui confère à la face principale comportant ledit filtre les propriétés suivantes :

- un facteur moyen de réflexion dans le bleu ($R_{m,B}$) sur une gamme de longueurs d'onde allant de 420 nanomètres à 450 nanomètres qui est supérieur ou égal à 10 %, de préférence supérieur ou égal à 20 %, mieux supérieur ou égal à 30 %, pour un angle d'incidence compris entre 0° et 15°,
- une courbe de réflectivité spectrale pour un angle d'incidence compris entre 0° et 15°, cette courbe de réflectivité ayant :

    - un maximum de réflectivité à une longueur d'onde inférieure à 435 nanomètres, et
    - une largeur à mi-hauteur (FWHM) supérieure ou égale à 70 nanomètres, de préférence supérieure ou égale à 75 nm, et **caractérisée en ce que**

- pour un angle d'incidence $\theta$ compris entre 0° et 15° et pour un angle d'incidence $\theta'$ compris entre 30° et 45°, un paramètre $\Delta(\theta,\theta')$ défini par la relation $\Delta(\theta,\theta') = 1 - [ R_{\theta'}(435\ nm) / R_{\theta}(435\ nm) ]$, tel que ce paramètre $\Delta(\theta,\theta')$ soit supérieur ou égal à 0,5, où

    - $R_{\theta}(435\ nm)$ représente la valeur de la réflectivité de la face principale comportant ledit filtre à la longueur d'onde de 435 nanomètres pour l'angle d'incidence $\theta$, et
    - $R_{\theta'}(435\ nm)$ représente la valeur de la réflectivité de la face principale comportant ledit filtre à la longueur d'onde de 435 nanomètres pour l'angle d'incidence $\theta'$ et

- pour un angle d'incidence compris entre 0° et 15°, un paramètre $\Delta_{spectral}$ défini par la relation $\Delta_{spectral} = 1 - [ R_{0°-15°}(480\ nm) / R_{0°-15°}(435\ nm) ]$, tel que ce paramètre $\lambda_{spectral}$ soit supérieur ou égal à 0,8, où

    - $R_{0°-15°}(480\ nm)$ représente la valeur de la réflectivité de la face principale avant à la longueur d'onde de 480 nanomètres à l'incidence considérée, et
    - $R_{0°-15°}(435\ nm)$ représente la valeur de la réflectivité de la face principale avant à la longueur d'onde de 435 nanomètres à l'incidence considérée.

18. Lentille ophtalmique selon la revendication 17, dans laquelle le paramètre $\Delta(\theta,\theta')$ est défini pour un angle d'incidence $\theta = 15°$ et un angle d'incidence $\theta' = 45°$.

19. Lentille ophtalmique selon la revendication 18, dans laquelle le paramètre $\Delta_{spectral}$ est défini pour un angle d'incidence de 15°.

20. Lentille ophtalmique selon l'une des revendications 17 à 19, dans laquelle le maximum de réflectivité est à une longueur d'onde supérieure ou égale à 350 nm, mieux supérieure ou égale à 360 nm.

21. Lentille ophtalmique selon l'une des revendications 17 à 20, dans laquelle la largeur à mi-hauteur est inférieure ou égale à 150 nanomètres, de préférence inférieure ou égale à 120 nanomètres, mieux inférieure ou égale à 110 nm.

**Patentansprüche**

1. Ophthalmische Linse, die eine vordere Hauptseite und eine hintere Hauptseite aufweist, wobei mindestens eine der Hauptseiten ein Filter umfasst, das der das Filter umfassenden Hauptseite die folgenden Eigenschaften verleiht:

   - einen mittleren Reflexionsfaktor im Blau ($R_{m,B}$) über einen Wellenlängenbereich von 420 Nanometer bis 450 Nanometer, der größer als oder gleich 5 % bei einem Einfallswinkel zwischen 0° und 15° ist,
   - eine spektrale Reflektivitätskurve bei einem Einfallswinkel zwischen 0° und 15°, wobei diese Reflektivitätskurve besitzt:

     - ein Reflektivitätsmaximum bei einer Wellenlänge unter 435 Nanometer, und
     - eine Halbwertsbreite (FWHM) größer als oder gleich 80 Nanometer, **dadurch gekennzeichnet, dass**
     - der Wert der Reflektivität am Reflektivitätsmaximum der das Filter umfassenden Hauptseite bei einem Einfallswinkel von 15° mindestens 1,5 Mal größer ist als der Wert der Reflektivität dieser selben Hauptseite bei der Wellenlänge von 435 nm und bei einem Einfallswinkel von 15°, und
     - bei einem Einfallswinkel $\theta$ zwischen 0° und 15° und bei einem Einfallswinkel $\theta'$ zwischen 30° und 45° einen durch die Gleichung $\Delta(\theta, \theta') = 1 - [R_{\theta'}(435\,nm)\,/\,R_{\theta}(435\,nm)]$ definierten Parameter $\Delta(\theta,\theta')$ derart, dass dieser Parameter $\Delta(\theta,\theta')$ größer als oder gleich 0,6 ist, wobei
     - $R_{\theta}(435\,nm)$ den Wert der Reflektivität der das Filter umfassenden Hauptseite bei der Wellenlänge von 435 Nanometer bei dem Einfallswinkel $\theta$ darstellt, und
     - $R_{\theta'}(435\,nm)$ den Wert der Reflektivität der das Filter umfassenden Hauptseite bei der Wellenlänge von 435 Nanometer bei dem Einfallswinkel $\theta'$ darstellt.

2. Ophthalmische Linse nach Anspruch 1, wobei der Parameter $\Delta(\theta,\theta')$ für einen Einfallswinkel $\theta = 15°$ und einen Einfallswinkel $\theta' = 45°$ definiert ist.

3. Ophthalmische Linse nach Anspruch 1 oder 2, wobei der mittlere Reflexionsfaktor im Blau ($R_{m,B}$) größer als oder gleich 10 %, besser größer als oder gleich 20 % und noch besser größer oder gleich 30 % bei einem Einfallswinkel zwischen 0° und 15° ist.

4. Ophthalmische Linse nach einem der Ansprüche 1 bis 3, wobei das Reflektivitätsmaximum bei einer Wellenlänge kleiner als oder gleich 410 nm, besser kleiner als oder gleich 400 nm und noch besser kleiner als oder gleich 390 nm liegt.

5. Ophthalmische Linse nach einem der Ansprüche 1 bis 4, wobei das Reflektivitätsmaximum bei einer Wellenlänge größer oder gleich 350 nm, besser größer oder gleich 360 nm liegt.

6. Ophthalmische Linse nach einem der Ansprüche 1 bis 5, wobei die Halbwertsbreite größer als oder gleich 90 Nanometer, vorzugsweise größer als oder gleich 100 Nanometer ist.

7. Ophthalmische Linse nach einem der Ansprüche 1 bis 6, wobei die Halbwertsbreite kleiner als oder gleich 150 Nanometer, vorzugsweise kleiner als oder gleich 120 Nanometer, besser kleiner als oder gleich 110 nm ist.

8. Ophthalmische Linse nach einem der Ansprüche 1 bis 7, wobei der mittlere Lichtreflexionsfaktor ($R_v$) auf der das Filter umfassenden Hauptseite der ophthalmischen Linse kleiner oder gleich 2,5 %, bevorzugt kleiner oder gleich 1,5 % ist.

9. Ophthalmische Linse nach einem der Ansprüche 1 bis 7, wobei der mittlere Lichtreflexionsfaktor ($R_v$) auf jeder der Hauptseiten der ophthalmischen Linse kleiner als oder gleich 2,5%, bevorzugt kleiner als oder gleich 1,5% ist.

10. Ophthalmische Linse nach einem der Ansprüche 1 bis 9, wobei das Filter ein Interferenzfilter ist.

11. Ophthalmische Linse nach Anspruch 10, wobei das Filter eine Anzahl von Schichten kleiner als oder gleich 11, bevorzugt von 2 bis 10 Schichten und noch stärker bevorzugt von 4 bis 9 Schichten umfasst.

12. Ophthalmische Linse nach einem der Ansprüche 1 bis 11, wobei das Filter eine Gesamtdicke kleiner als oder gleich 700 Nanometer, vorzugsweise kleiner als oder gleich 600 Nanometer aufweist.

13. Ophthalmische Linse nach einem der Ansprüche 1 bis 12, **dadurch gekennzeichnet, dass** das Verhältnis [$R_{15°}$(435 nm) - R$_{15°}$ (480 nm)] / R$_{15°}$ (435 nm) größer als oder gleich 0,8 ist, wobei R$_{15°}$ (435 nm) und R$_{15°}$(480 nm) die Reflektivität der das Filter umfassenden Hauptseite bei 435 nm beziehungsweise bei 480 nm bei einem Einfallswinkel von 15° auf dieser Hauptseite darstellen.

14. Ophthalmische Linse nach einem der Ansprüche 1 bis 13, **dadurch gekennzeichnet, dass** der Wert der Reflektivität am Reflektivitätsmaximum der das Filter umfassenden Hauptseite bei einem Einfallswinkel von 15° mindestens 2 Mal größer ist als der Wert der Reflektivität dieser selben Hauptseite bei der Wellenlänge von 435 nm und bei einem Einfallswinkel von 15°.

15. Ophthalmische Linse nach einem der Ansprüche 1 bis 14, wobei das Filter auf der vorderen Hauptseite der ophthalmischen Linse gebildet ist.

16. Ophthalmische Linse nach Anspruch 15, wobei die hintere Hauptseite der ophthalmischen Linse eine im UV wirksame Antireflexbeschichtung umfasst, die einen mittleren Reflexionsfaktor im UV R$_{UV}$ auf der hinteren Hauptseite der ophthalmischen Linse bei Wellenlängen zwischen 280 nm und 380 nm, gewichtet durch die in der Norm ISO 13666:1998 definierte Funktion W($\lambda$), kleiner als oder gleich 7 % bei einem Einfallswinkel von 30° und bei einem Einfallswinkel von 45° hat, wobei der mittlere Reflexionsfaktor im UV R$_{UV}$ definiert ist durch die Gleichung:

$$R_{UV} = \frac{\int_{280}^{380} W(\lambda).R(\lambda).d\lambda}{\int_{280}^{380} W(\lambda).d\lambda}$$

wobei R($\lambda$) die spektrale Reflektivität auf der hinteren Hauptseite der ophthalmischen Linse bei der betrachteten Wellenlänge bezeichnet und W($\lambda$) eine Gewichtungsfunktion bezeichnet, die gleich dem Produkt aus der spektralen solare Bestrahlungsstärke Es($\lambda$) und aus der relativen spektralen Funktion mit der Wirksamkeit S($\lambda$) ist.

17. Ophthalmische Linse, die eine vordere Hauptseite und eine hintere Hauptseite aufweist, wobei mindestens eine der Hauptseiten ein Filter umfasst, das der das Filter umfassenden Hauptseite die folgenden Eigenschaften verleiht:

- einen mittleren Reflexionsfaktor im Blau (R$_{m,B}$) über einen Wellenlängenbereich von 420 Nanometer bis 450 Nanometer, der größer als oder gleich 10 %, vorzugsweise größer als oder gleich 20 %, besser größer als oder gleich 30 % bei einem Einfallswinkel zwischen 0° und 15° ist,
- eine spektrale Reflektivitätskurve bei einem Einfallswinkel zwischen 0° und 15°, wobei diese Reflektivitätskurve besitzt:

- ein Reflektivitätsmaximum bei einer Wellenlänge unter 435 Nanometer, und
- eine Halbwertsbreite (FWHM) größer als oder gleich 70 Nanometer, vorzugsweise größer als oder gleich 75 nm, und **dadurch gekennzeichnet, dass**
- bei einem Einfallswinkel $\theta$ zwischen 0° und 15° und bei einem Einfallswinkel $\theta'$ zwischen 30° und 45° einen durch die Gleichung $\Delta(\theta,\theta') = 1 - [R_{\theta'}(435 \text{ nm}) / R_\theta(435 \text{ nm})]$ definierten Parameter $\Delta(\theta,\theta')$ derart, dass dieser Parameter $\Delta(\theta,\theta')$ größer als oder gleich 0,5 ist, wobei
- R$_\theta$(435 nm) den Wert der Reflektivität der das Filter umfassenden Hauptseite bei der Wellenlänge von 435 Nanometer bei dem Einfallswinkel $\theta$ darstellt, und
- R$_{\theta'}$ (435 nm) den Wert der Reflektivität der das Filter umfassenden Hauptseite bei der Wellenlänge von 435 Nanometer bei dem Einfallswinkel $\theta'$ darstellt und
- bei einem Einfallswinkel zwischen 0° und 15° einen durch die Gleichung $\Delta_{spectral} = 1 - [R_{0°-15°} (480 \text{ nm}) / R_{0°-15°} (435 \text{ nm})]$ definierten Parameter $\Delta_{spectral}$ derart, dass dieser Parameter $\Delta_{spectral}$ größer als oder gleich 0,8 ist, wobei
- R$_{0°-15°}$ (480 nm) den Wert der Reflektivität der vorderen Hauptseite bei der Wellenlänge von 480 Nanometer bei dem betrachteten Einfallswinkel darstellt, und
- R$_{0°-15°}$ (435 nm) den Wert der Reflektivität der vorderen Hauptseite bei der Wellenlänge von 435 Nanometer bei dem betrachteten Einfallswinkel darstellt.

18. Ophthalmische Linse nach Anspruch 17, wobei der Parameter $\Delta(\theta,\theta')$ für einen Einfallswinkel $\theta$ = 15° und einen

Einfallswinkel θ' = 45° definiert ist.

19. Ophthalmische Linse nach Anspruch 18, wobei der Parameter $\Delta_{spectral}$ für einen Einfallswinkel von 15° definiert ist.

20. Ophthalmische Linse nach einem der Ansprüche 17 bis 19, wobei das Reflektivitätsmaximum bei einer Wellenlänge größer als oder gleich 350 nm, besser größer als oder gleich 360 nm liegt.

21. Ophthalmische Linse nach einem der Ansprüche 17 bis 20, wobei die Halbwertsbreite kleiner als oder gleich 150 Nanometer, vorzugsweise kleiner als oder gleich 120 Nanometer, besser kleiner als oder gleich 110 nm ist.

**Claims**

1. An ophthalmic lens having a front main face and a back main face, at least one of both main faces comprising a filter, which provides the main face comprising said filter with the following properties:

   - an average blue reflectance factor ($R_{m,B}$) within a wavelength range of from 420 nanometers to 450 nanometers, which is higher than or equal to 5%, for an angle of incidence ranging from 0° to 15°,
   - a spectral reflectivity curve for an angle of incidence ranging from 0° to 15°, this reflectivity curve having:

      - a maximum reflectivity at a wavelength of less than 435 nanometers, and
      - a full width at half maximum (FWHM) higher than or equal to 80 nanometers, **characterized in that**

   - the reflectivity value at the maximum level of reflectivity of the main face comprising said filter, for an angle of incidence of 15°, is at least 1.5 times higher than the reflectivity value of the same main face, at a wavelength of 435 nm and for an angle of incidence of 15°, and
   - for an angle of incidence θ ranging from 0° to 15° and for an angle of incidence θ' ranging from 30° to 45°, a parameter $\Delta(\theta,\theta')$ defined by the relation $\Delta(\theta,\theta') = 1 - [R_{\theta'}(435\,nm) / R_{\theta}(435\,nm)]$, in such a way that this parameter $\Delta(\theta,\theta')$ is higher than or equal to 0.6, where

      - $R_{\theta}(435\,nm)$ represents the reflectivity value of the main face comprising said filter at a 435 nanometer-wavelength for the angle of incidence θ, and
      - $R_{\theta'}(435\,nm)$ represents the reflectivity value of the main face comprising said filter at a 435 nanometer-wavelength for the angle of incidence θ'.

2. An ophthalmic lens according to claim 1, wherein the parameter $\Delta(\theta,\theta')$ is defined for an angle of incidence θ = 15° and for an angle of incidence θ' = 45°.

3. An ophthalmic lens according to claim 1 or 2, wherein the average blue reflectance factor ($R_{m,B}$) is higher than or equal to 10%, more preferably higher than or equal to 20%, and even more preferably higher than or equal to 30% for an angle of incidence ranging from 0° to 15°.

4. An ophthalmic lens according to any one of claims 1 to 3, wherein the maximum reflectivity is observed at a wavelength lower than or equal to 410 nm, more preferably lower than or equal to 400 nm, and even more preferably lower than or equal to 390 nm.

5. An ophthalmic lens according to any one of claims 1 to 4, wherein the maximum reflectivity is observed at a wavelength higher than or equal to 350 nm, more preferably higher than or equal to 360 nm.

6. An ophthalmic lens according to any one of claims 1 to 5, wherein the full width at half maximum is higher than or equal to 90 nanometers, preferably higher than or equal to 100 nanometers.

7. An ophthalmic lens according to any one of claims 1 to 6, wherein the full width at half maximum is lower than or equal to 150 nanometers, preferably lower than or equal to 120 nanometers, more preferably lower than or equal to 110 nm.

8. An ophthalmic lens according to any one of claims 1 to 7, wherein the average luminous reflectance factor ($R_v$) on the main face of the ophthalmic lens comprising the filter is lower than or equal to 2.5%, preferably lower than or equal to 1.5%.

9. An ophthalmic lens according to any one of claims 1 to 7, wherein the average luminous reflectance factor ($R_v$) on each of the main faces of the ophthalmic lens is lower than or equal to 2.5%, preferably lower than or equal to 1.5%.

10. An ophthalmic lens according to any one of claims 1 to 9, wherein the filter is an interference filter.

11. An ophthalmic lens according to claim 10, wherein the filter comprises a number of layers lower than or equal to 11, preferably ranging from 2 to 10 layers, and more preferably from 4 to 9 layers.

12. An ophthalmic lens according to any one of claims 1 to 11, wherein the filter has a total thickness lower than or equal to 700 nanometers, preferably lower than or equal to 600 nanometers.

13. An ophthalmic lens according to any one of claims 1 to 12, **characterized in that** the ratio [ $R_{15°}$(435 nm) - $R_{15°}$(480 nm) ] / $R_{15°}$(435 nm) is higher than or equal to 0.8, where $R_{15°}$(435 nm) and $R_{15°}$(480 nm) represent respectively the reflectivity of the main face comprising said filter at 435 nm and at 480 nm, for an angle of incidence of 15° on this main face.

14. An ophthalmic lens according to any one of claims 1 to 13, **characterized in that** the reflectivity value at the maximum level of reflectivity of the main face comprising said filter, for an angle of incidence of 15°, is at least 2 times higher than the reflectivity value of the same main face, at a wavelength of 435 nm and for an angle of incidence of 15°.

15. An ophthalmic lens according to any one of claims 1 to 14, wherein the filter is formed on the front main face of the ophthalmic lens.

16. An ophthalmic lens according to claim 15, wherein the back main face of the ophthalmic lens comprises an antireflection coating efficient within the UV region having an average reflectance factor within the UV region $R_{UV}$ on the back main face of the ophthalmic lens, for wavelengths ranging from 280 nm to 380 nm, weighted by the function $W(\lambda)$ defined in the standard ISO 13666:1998, lower than or equal to 7%, for an angle of incidence of 30° and for an angle of incidence of 45°, the average reflectance factor within the UV region $R_{UV}$ being defined by the relation:

$$R_{UV} = \frac{\int_{280}^{380} W(\lambda).R(\lambda).d\lambda}{\int_{280}^{380} W(\lambda).d\lambda}$$

wherein $R(\lambda)$ represents the spectral reflectivity on the back main face of the ophthalmic lens at the relevant wavelength, and $W(\lambda)$ represents a weighting function that is equal to the product of solar spectral irradiance $Es(\lambda)$ and relative spectral efficiency function $S(\lambda)$.

17. An ophthalmic lens having a front main face and a back main face, at least one of both main faces comprising a filter, which provides the main face comprising said filter with the following properties:

   - an average blue reflectance factor ($R_{m,B}$) within a wavelength range of from 420 nanometers to 450 nanometers, which is higher than or equal to 10%, preferably higher than or equal to 20%, more preferably higher than or equal to 30%, for an angle of incidence ranging from 0° to 15°,
   - a spectral reflectivity curve for an angle of incidence ranging from 0° to 15°, this reflectivity curve having:

      - a maximum reflectivity at a wavelength of less than 435 nanometers, and
      - a full width at half maximum (FWHM) higher than or equal to 70 nanometers, preferably higher than or equal to 75 nm, and **characterized in that**

   - for an angle of incidence θ ranging from 0° to 15° and for an angle of incidence θ' ranging from 30° to 45°, a parameter $\Delta(\theta,\theta')$ defined by the relation $\Delta(\theta,\theta') = 1 - [ R_{\theta'}(435\,nm) / R_{\theta}(435\,nm)]$, in such a way that this parameter $\Delta(\theta,\theta')$ is higher than or equal to 0.5, where

      - $R_{\theta}$(435 nm) represents the reflectivity value of the main face comprising said filter at a 435 nanometer-wavelength for the angle of incidence $\theta$, and

- $R_{\theta'}$(435 nm) represents the reflectivity value of the main face comprising said filter at a 435 nanometer-wavelength for the angle of incidence $\theta'$ and

- for an angle of incidence ranging from 0° to 15°, a parameter $\Delta_{spectral}$ defined by the relation $\Delta_{spectral} = 1 - [R_{0°-15°}$(480 nm) / $R_{0°-15°}$(435 nm)], in such a way that this parameter $\Delta_{spectral}$ is higher than or equal to 0.8, where

- $R_{0°-15°}$(480 nm) represents the reflectivity value of the front main face at a 480 nanometer-wavelength for the relevant incidence, and

- $R_{0°-15°}$(435 nm) represents the reflectivity value of the front main face at a 435 nanometer-wavelength for the relevant incidence.

18. An ophthalmic lens according to claim 17, wherein the parameter $\Delta(\theta,\theta')$ is defined for an angle of incidence $\theta$ = 15° and an angle of incidence $\theta'$ = 45°.

19. An ophthalmic lens according to claim 17, wherein the parameter $\Delta_{spectral}$ is defined for an angle of incidence of 15°.

20. An ophthalmic lens according to any one of claims 17 to 19, wherein the maximum reflectivity is observed at a wavelength higher than or equal to 350 nm, more preferably higher than or equal to 360 nm.

21. An ophthalmic lens according to any one of claims 17 to 20, wherein the full width at half maximum is lower than or equal to 150 nanometers, preferably lower than or equal to 120 nanometers, more preferably lower than or equal to 110 nm.

Figure 1

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008024414 A
- FR 2734827
- EP 2011072386 W
- EP 0614957 A
- US 7678464 B
- EP 614957 A
- WO 2008107325 A

**Littérature non-brevet citée dans la description**

- **KITCHEL E.** The effects of blue light on ocular health. *Journal of Visual Impairment and Blindness*, 2000, vol. 94 (6)
- **GLAZER-HOCKSTEIN et al.** *Retina*, 2006, vol. 26 (1), 1-4
- **ALGVERE P.V. et al.** Age-Related Maculopathy and the Impact of the Blue Light Hazard. *Acta Ophthalmo. Scand.*, 2006, vol. 84, 4-15
- **TOMANY S.C. et al.** Sunlight and the 10-Year Incidence of Age-Related Maculopathy. The Beaver Dam Eye Study. *Arch Ophthalmol.*, 2004, vol. 122, 750-757
- Thin Film Processes. Academic Press, 1978
- Thin Film Processes II. Academic Press, 1991